# EUROPEAN PATENT APPLICATION

(11) **EP 4 682 167 A1**
(43) Date of publication of application: **21.01.2026**
(21) Application number: 24189467.4
(22) Date of filing: 18.07.2024
(51) Int. Cl.: C07K 16/28, A61P 33/06, A61K 39/395

(54) **THERAPEUTIC VARIABLE DOMAIN OF HEAVY CHAIN (VHH) ANTIBODIES AGAINST BASIGIN (CD147) AND THEIR USE FOR BLOCKING PLASMODIUM FALCIPARUM ENTRY INTO HUMAN ERYTHROCYTES**

(71) Applicant: Max-Planck-Gesellschaft zur Förderung der Wissenschaften e.V., 80539 München (DE)
(72) Inventor: The designation of the inventor has not yet been filed
(74) Representative: Weickmann & Weickmann PartmbB

(57) **Abstract**

The present invention pertains to the fields of antibody technology, medicine, pharmacology, infection biology, and virology. More specifically, the present disclosure provides VHH antibodies that prevent erythrocyte entry of and infection by *Plasmodium falciparum.*

## Description

### Field of the invention

The present invention pertains to the fields of antibody technology, medicine, pharmacology, infection biology, and virology. More specifically, the present disclosure provides VHH antibodies that prevent erythrocyte entry of and infection by *Plasmodium falciparum.*

### Background

Malaria is the deadliest parasitic disease, associated with more than half a million death annually, mostly of children (Phillips et al., 2017). *Plasmodium falciparum* is the causative agent of tropical malaria, by far the deadliest form of the disease. Available treatment schemes include derivatives of artemisinin, quinine, and atovaquone. They are effective in principle, but two major obstacles can impair successful therapy: the emergence of resistant plasmodium strains (Duffey et al., 2021; Wicht et al., 2020) and often the late treatment start, when severe malaria is already manifest (Mousa et al., 2020).

Life-threatening forms of malaria are characterized by parasitized red blood cells (RBCs or erythrocytes) adhering to blood vessels, causing decreased blood supply and hemorrhage in multiple organs, including the brain (Hadjilaou et al., 2023). Even when reducing the viability of plasmodia, current therapeutics leave parasitized RBCs behind for extended periods after treatment start (Hughes et al., 2010), and this may contribute to prolonged symptoms and death.

Available vaccines protect at 55% (Beeson et al., 2022). This protection rate may increase with ongoing trials using improved versions of the vaccine RTS,S, although some concerns concerning safety were raised (Björkman et al., 2023). Importantly, however, these vaccines only raise antibodies against the circumsporozoite protein (CSP) of *Plasmodium falciparum* (Zavala, 2022). Antibodies against CSP can only neutralize the sporozoites that travel from the mosquito to the liver. Thus, in cases where the vaccine does not fully prevent liver cell invasion by sporozoites, the subsequent blood stages of malaria are not prevented and conceivably not even diminished.

Upon transmission of sporozoites through mosquito bites, the first stage of parasite expansion occurs in liver cells, releasing merozoites. These then invade RBCs, where they further proliferate as ring stages, trophozoites and schizonts, followed by egress of fresh merozoites and invasion of new RBCs (Phillips et al., 2017). Thus, the ability of merozoites to invade RBCs is crucial for the propagation of plasmodia and for the manifestation of the disease.

The entry of plasmodia to RBCs is mediated through a number of molecular interactions between surface molecules on the parasites and their host cells (Cowman et al., 2017; Kumar & Tolia, 2019). Rhoptry proteins form an apical structure on the parasite and mediate entry. In particular, RBC invasion requires the interaction of the plasmodial PfRH5 (*Plasmodium falciparum* reticulocyte-binding protein homolog 5) with the RBC surface receptor basigin (also known as CD147 and EMMPRIN). Genetic deletion of basigin in precursor cells renders RBCs completely refractory to invasion by *Plasmodium falciparum* (Kanjee et al., 2017; Satchwell et al., 2019). The importance of this interaction for host invasion is further emphasized by the rapid co-evolution of basigin and PfRH5 in humans and great apes along with their corresponding Plasmodium species (Galaway et al., 2019; Wanaguru et al., 2013). Apparently, plasmodia imposed an effective evolutionary pressure on basigin through PfRH5 binding. This interaction is thus an ideal target for blocking the infection cycle.

Antibodies to PfRH5 (Douglas et al., 2014; Williams et al., 2012) and its interaction partners CyRPA (Chen et al., 2017; Reddy et al., 2015) as well as Plasmodium thrombospondin-related apical merozoite protein (PTRAMP) and cysteine-rich small secreted protein (CSS) (Scally et al., 2022) were all found to reduce or abolish the propagation of plasmodia in erythrocytes. PfRH5-based experimental vaccines were also capable of eliciting antibodies with this activity (Alanine et al., 2019; Douglas et al., 2015; Douglas et al., 2011). However, antibodies against PfRH5 and its plasmodial interaction partners were not successfully developed as therapeutics so far. Their efficacy might be diminished by the short exposure of merozoites - typical times between egress and re-invasion are estimated to be 10 minutes or less (Boyle et al., 2010; Gilson & Crabb, 2009). Moreover, the exposure of PfRH5 requires protease-mediated cleavage of a precursor protein (Favuzza et al., 2020; Pino et al., 2017; Triglia et al., 2023) and may thus occur during even shorter periods. Thus, the therapeutic efficacy of antibodies against PfRH5 might be limited by kinetic competition between them and basigin for PfRH5 binding.

An alternative approach for preventing the entry of plasmodia consists of targeting basigin by antibodies. This also reduces the risk of escape mutations within PfRH5 that might destroy the efficacy of antibodies directed against it. A monoclonal antibody to basigin was found effective in preventing the spread of *Plasmodium falciparum* (Zenonos et al., 2015). However, full-sized antibodies to basigin might induce opsonisation and complement activation towards RBCs, causing hemolysis. Thus, for clinical applications, it appears more appropriate to develop smaller antibody fragments that do not contain an Fc domain, or even single-domain antibodies to basigin.

### Summary of the invention

The present disclosure provides single-domain VHH antibodies directed to basigin, which bind basigin at the erythrocyte surfaces and block interaction with *Plasmodium falciparum* RH5 (PfRH5). These VHH antibodies are suitable for diagnostic, prophylactic and/or therapeutic applications.

In certain embodiments, the VHH antibody is in a monovalent form, e.g., as a single VHH domain or as a fusion to a heterologous protein such as serum albumin useable in a wide range of formats. In certain embodiments, the VHH antibody is in a multivalent form, e.g., as bivalent Fc-fusion. In certain embodiments, the VHH antibody is a hetero-multimeric VHH antibody comprising two or more different VHH antibody units.

The use in a monovalent format is possible due to their very high affinity. In certain embodiments, the VHH antibodies have - in the monovalent format - a target affinity in picomolar or even low picomolar range. In certain embodiments, the VHH antibodies block infection of RBCs by *P. falciparum* parasites with high potency.

A first aspect of the present disclosure is a VHH antibody directed against basigin as described herein. Preferred VHH antibodies of the present invention, their designations, binding characteristics, i.e., their targeted epitope, target affinity, PfRH5 competition and block of parasite invasion as well as their thermostabilities are listed in the following Table 1:

### Table 1

**Table 1: Anti-basigin VHHs, their thermostability, affinities (expressed as KD values) and inhibitory properties.**

| **VHH** | **Class** | **Epitope** | **Affinity (pM)** | **Thermostability (°C)** | | **PfRH5 competition** | **Infection block** |
|---|---|---|---|---|---|---|---|
| | | | | **-DTT** | **+DTT** | | |
| Bm7D12 | Bm7D12 | 1 | ≤10 | >95 | >95 | Yes | Yes |
| Bm7H08 | Bm7D12 | 1 | ≤10 | >95 | >95 | Yes | Yes |
| Bm7G12 | Bm7D12 | 1 | 20 | >95 | >95 | Yes | Yes |
| Bm7E11 | Bm7D12 | 1 | 50 | >95 | >95 | Yes | Yes |
| Bm7F03 | Bm7D12 | 1 | 50 | >95 | >95 | Yes | Yes |
| Bm7G08 | Bm7D12 | 1 | ≤10 | >95 | 51 | Yes | Yes |
| Bm27D04 | Bm7D12 | 1 | 150 | >95 | | Yes | Yes |
| Bm27H02 | Bm7D12 | 1 | 90 | >95 | | Yes | Yes |
| Bm8D12 | Bm8D12 | 1 | ≤10 | >95 | >95 | Yes | Yes |
| Bm8B10 | Bm8D12 | 1 | ≤10 | >95 | 60 | Yes | Yes |
| Bm8D09 | Bm8D12 | 1 | ≤10 | 65 | 59 | Yes | Yes |
| Bm8H12 | Bm8D12 | 1 | ≤10 | >95 | >95 | Yes | Yes |
| Bm8C06 | Bm8D12 | 1 | 50 | 61 | 55 | Yes | No |
| Bm8H07 | Bm8D12 | 1 | 170 | >95 | >95 | Yes | Yes |
| Bm8G09 | Bm8D12 | 1 | 2000 | >95 | 55 | Yes | No |
| Bm8G10 | Bm8G10 | 1 | 60 | >95 | 33 | Yes | Yes |
| Bm8F05 | Bm8G10 | 1 | ≤10 | >95 | 38 | Yes | Yes |
| Bm8H10 | Bm8H10 | 1 | ≤10 | >95 | 37 | Yes | Yes |
| Bm8G06 | Bm8G06 | 1 | 65 | 63 | 37 | Yes | Yes |
| Bm8G07 | Bm8G07 | 2 | 50 | 61 | 48 | No | No |
| Bm8G12 | Bm8G12 | 2 | 10 | >95 | >95 | No | No |
| Bm27C04 | Bm8G12 | 2 | ≤10 | | | No | No |
| Bm27C06 | Bm8G12 | 2 | 10 | | | No | No |
| Bm27E06 | Bm27E06 | 1 | 70 | | | Yes | Yes |
| Bm27H03 | Bm27E06 | 1 | ≤10 | | | Yes | Yes |
| Bm27C11 | Bm27C11 | 3 | 300 | >95 | >95 | No | No |
| Bm27D06 | Bm27C11 | 3 | ≤10 | >95 | >95 | No | No |

A list of complete VHH sequences of VHH antibodies is shown at the end of the specification.

A further aspect of the present invention relates to a set of two or more different VHH antibodies, wherein at least one VHH antibody is as described above.

AVHH antibody described above is suitable for use in medicine, e.g., human medicine, particularly for use in therapy, e.g., in the prevention, treatment and/or mitigation of a condition caused by, associated with and/or accompanied by an infection with P. *falciparum* or another pathogen that invades mammalian cells by interaction with basigin, or in diagnostics, e.g., for labelling and/or immobilization of erythrocytes for diagnostic or research purposes.

Still a further aspect of the invention relates to a nucleic acid molecule encoding a VHH antibody as described above, particularly in operative linkage with a heterologous expression control sequence, a vector comprising said nucleic acid molecule or a recombinant cell or non-human organism transformed or transfected with said nucleic acid molecule or said vector.

Still a further aspect of the invention relates to a method for the recombinant production of a VHH antibody as described above, comprising cultivating a cell or an organism as described above in a suitable medium and obtaining the VHH antibody from the cell or organism or from the medium.

Still a further aspect of the invention relates to a method for the prevention, treatment and/or mitigation of a condition caused by, associated with and/or accompanied by an activity of basigin, comprising administering an effective dose of the VHH antibody as described above or the set of at least two different VHH antibodies as described above to a subject in need thereof, particularly to a human subject in need thereof.

In certain embodiments, the condition is malaria.

### Table 2

**Table 2: Anti-basigin VHHs and their CDR regions. Note that CDRs include here also variable residues that flank the actual CDR loops**

| VHH | SEQ ID NO. | CDR1 | SEQ ID NO. | CDR2 | SEQ ID NO. | CDR3 | SEQ ID NO. |
|---|---|---|---|---|---|---|---|
| Bm7D12 | 5 | GLGSYGVGWFR | 6 | GVSCMSTSGRVSYDAD | 7 | AKVRYLAYGDKVCLADEDTIW | 8 |
| Bm7H08 | 9 | GLGNYGIGWFH | 10 | GVSCMSTSGRVTYDVD | 11 | AKVRY LAY GDKVCLADENASW | 12 |
| Bm7G12 | 13 | GLGNYGIGWFH | 14 | GVSCMSTSGRVTYDVD | 15 | AKVRYLAYGDKVCLADENASW | 16 |
| Bm7E11 | 17 | GLGNYGIGWFR | 18 | GVSCMSSSGRVTYNVD | 19 | AKVRYLAYGDKVCLADENASW | 20 |
| Bm7F03 | 21 | GLGNYGIGWFR | 22 | GVSCMSSSGRVTYNVD | 23 | AKVRYLAYGDKVCLADENAYW | 24 |
| Bm7G08 | 25 | GLGNYGIGWFY | 26 | GVSCMSTSGRVTYDVD | 27 | AKVRYLAYGDKVCLADENASW | 28 |
| Bm27D04 | 29 | GLGKSGIGWFH | 30 | GVSCMSTSGRVSYDAD | 31 | ARVHYLAYGDRICLADDNASW | 32 |
| Bm27H02 | 33 | GLGSYGIGWFR | 34 | GVSCMSTSGRVSYDAD | 35 | AKVRYLAYGDKVCLADEYTSW | 36 |
| Bm8D12 | 37 | GRSFSNYAMGWFR | 38 | FVSATSWSDGTNYYAEASE | 39 | AARGSTGWATKEAQDYVYW | 40 |
| Bm8B10 | 41 | GRTFTNVAMAWFR | 42 | FVSGISWTGGTSYYAEADD | 43 | AARESAGWATKKAQDYVYW | 44 |
| Bm8D09 | 45 | GRTFTNYAMAWFR | 46 | FVSGISWTGGTSYYAEADD | 47 | AARESAGWATKKAQDYVYW | 48 |
| Bm8H12 | 49 | GRTFENYAMGWFR | 50 | FVSATSWTGGTNYYAEASE | 51 | AVRGSTGWATKRAQEYDYW | 52 |
| Bm8C06 | 53 | GRTFTNYAMGWFR | 54 | FVSATSWTGDTNYYAEATE | 55 | AARGSAGWATQQAQDYVYW | 56 |
| Bm8H07 | 57 | EGLFSNYAMGWFR | 58 | FVSATSWNGGTNYYAEAAE | 59 | AARDSEGWATKNAQEYVYW | 60 |
| Bm8G09 | 61 | GRTFENYAMGWFR | 62 | FVSAISWTGGTNYVAETTE | 63 | AARGSTGWATKEAQDYVYW | 64 |
| Bm8G10 | 65 | GFTLDAYAIGWFR | 66 | RVSCIGSGGGAALYAD | 67 | AGDCSASAYSLK | 68 |
| Bm8F05 | 69 | GITLNQYAIGWFR | 70 | VLSCIGSGAGGTRYAD | 71 | AADCSGGPYSLR | 72 |
| Bm8H10 | 73 | GSTLDGYSIGWFR | 74 | GVGCISRSSANRSYKD | 75 | AAQETEDQHACYYYRGMDYW | 76 |
| Bm8G06 | 77 | GSTLDHFSIGWFR | 78 | GVGCISWSGRNRAYKD | 79 | AAQEAVDWPDACYYYAGMPYW | 80 |
| Bm8G07 | 81 | GFTLGRYPIGWYR | 82 | GVACITGDGDLVDYNY | 83 | AAQVSSRSLCSLLKPFGGMEYW | 84 |
| Bm8G12 | 85 | EGIFVNHGMGWYR | 86 | WVAIMFGTGGTKYQD | 87 | NQGSIW | 88 |
| Bm27C04 | 89 | EGIFVNHGMGWYH | 90 | WVAIMFGTGGTKYQD | 91 | NQGSIW | 92 |
| Bm27C06 | 93 | EGIRVNHGMGWYR | 94 | WVAIMFGTGGTKYQD | 95 | NQGSIW | 96 |
| Bm27E06 | 97 | RLTVDQYAIGWFR | 98 | RVSCIGAGGGAAIYAD | 99 | AGDCSASAYSLK | 100 |
| Bm27H03 | 101 | GLTVDAYAIGWFR | 102 | RVSCIGSGGGAAIYAD | 103 | AGDCSASAYSLK | 104 |
| Bm27C11 | 105 | GFTFSKHAMGWYR | 106 | LVATISNGGSRTNYAD | 107 | NTGWGAGTEQTACTMSEKFGALDAW | 108 |
| Bm27D06 | 109 | GFTFSKHAMGWYR | 110 | LVATISNGGSRTNYAD | 111 | NTGWGAGTEQTACTMSEKFGALDAW | 112 |

### Embodiments of the invention

In the following, specific embodiments of the invention are disclosed as follows:
1. A VHH antibody recognizing basigin.
2. The VHH antibody of embodiment 1, which recognizes human basigin.
3. The VHH antibody of embodiment 1 or 2, which recognizes human basigin isoform 2.
4. The VHH antibody of any one of embodiments 1-3, which recognizes the human basigin isoform 2 ectodomain.
5. The VHH antibody of any one of embodiments 1-4, which binds to the same or an overlapping epitope on basigin as the VHH antibody Bm7D12 having a VHH sequence as shown in SEQ. ID NO: 5.
6. The VHH antibody of any one of embodiments 1-4, which binds to the same or an overlapping epitope on basigin as the VHH antibody Bm8D12 having a VHH sequence as shown in SEQ. ID NO: 37.
7. The VHH antibody of any one of embodiments 1-4, which binds to the same or an overlapping epitope on basigin as the VHH antibody Bm8G10 having a VHH sequence as shown in SEQ. ID NO: 65.
8. The VHH antibody of any one of embodiments 1-4 which binds to the same or an overlapping epitope on basigin as the VHH antibody Bm8H10 having a VHH sequence as shown in SEQ. ID NO: 73.
9. The VHH antibody of any one of embodiments 1-4, which binds to the same or an overlapping epitope on basigin as the VHH antibody Bm8G07 having a VHH sequence as shown in SEQ. ID NO: 81.
10. The VHH antibody of any one of embodiments 1-4, which binds to the same or an overlapping epitope on basigin as the VHH antibody Bm8G12 having a VHH sequence as shown in SEQ. ID NO: 85.
11.The VHH antibody of any one of embodiments 1-4, which binds to the same or an overlapping epitope on basigin as the VHH antibody Bm27E06 having a VHH sequence as shown in SEQ. ID NO: 97.
12. The VHH antibody of any one of embodiments 1-4, which binds to the same or an overlapping epitope on basigin as the VHH antibody Bm27C11 having a VHH sequence as shown in SEQ. ID NO: 105.
13. The VHH antibody of embodiment 5 recognizing basigin which interacts with amino acid residues F27, T28, T29, V30, E31, D32, G34, S35, I37, S78, Q81, Q100, L101, H102, G103, P104, R106, and E129 of basigin in the numbering of basigin isoform 2 (UniProt P35613-2).
14. The VHH antibody of embodiment 6 recognizing basigin which interacts with amino acid residues T25, V26, F27, T28, T29, V30, E31, L33, G34, L38, T40, S42, N44, V131, P132, P133, Q164, S190, and K191 of basigin in the numbering of basigin isoform 2 (UniProt P35613-2).
15.A VHH antibody recognizing basigin, comprising
   (a) a CDR3 sequence as shown in SEQ. ID NO: 8, 12, 16, 20, 24, 28, 32, 36, 40, 44, 48, 52, 56, 60, 64, 68, 72, 76, 80, 84, 88, 92, 96, 100, 104, 108 or 112; or
   (b) a CDR3 sequence, which has an identity of at least 80%, at least 90% or at least 95% to a CDR3 sequence of (a).
16. The VHH antibody of any of the preceding embodiments, comprising
   (a) a combination of CDR1, CDR2 and CDR3 sequences as shown in SEQ. ID NO: 6-8, 10-12, 14-16, 18-20, 22-24, 26-28, 30-32, 34-36, 38-40, 42-44, 46-48, 50-52, 54-56, 58-60, 62-64, 66-68, 70-72, 74-76, 78-80, 82-84, 86-88, 90-92, 94-96, 98-100, 102-104, 106-108 or 110-112; or
   (b) a combination of CDR1, CDR2 and CDR3 sequences which has an identity of at least 80%, at least 90% or at least 95% to a combination of CDR1, CDR2 and CDR3 sequences of (a).
17. The VHH antibody of any one of the preceding embodiments, comprising
   (a) a VHH sequence as shown in SEQ. ID NO: 5, 9, 13, 17, 21, 25, 29, 33, 37, 41, 45, 49, 53, 57, 61, 65, 69, 73, 77, 81, 85, 89, 93, 97, 101, 105, or 109; or
   (b) a sequence, which has an identity of at least 80%, at least 90%, at least 95% or at least 99% to a VHH sequence of (a).
18. The VHH antibody of any one of the preceding embodiments, wherein the binding affinity expressed as dissociation constant K_{D} to basigin is about 500 pM or less, about 100 pM or less, or about 10 pM or less.
19. The VHH antibody of any one of the preceding embodiments, which inhibits the binding of PfRH5 to human basigin.
20. The VHH antibody of embodiment 19, which inhibits the entry of Plasmodia into erythrocytes.
21. The VHH antibody of any one of the preceding embodiments, which is stable, particularly thermostable, or hyperthermostable.
22. The VHH antibody of embodiment 21, which has a melting temperature of at least about 60°C, of at least 80°C or of at least about 95°C when measured under non-reducing conditions.
23. The VHH antibody of embodiment 21 or 22, which has a melting temperature of at least about 30°C, of at least about 50°C or of at least about 95°C when measured under reducing conditions.
24. The VHH antibody of any one of embodiments 1-23, which is selected from VHH antibody Bm7D12 having a VHH sequence as shown in SEQ. ID NO: 5 or a VHH antibody, which is a variant thereof.
25. The VHH antibody of any one of embodiments 1-23, which is selected from VHH antibody Bm7H08 having a VHH sequence as shown in SEQ. ID NO: 9 or a VHH antibody, which is a variant thereof.
26. The VHH antibody of any one of embodiments 1-23, which is selected from VHH antibody Bm7G12 having a VHH sequence as shown in SEQ. ID NO: 13 or a VHH antibody, which is a variant thereof.
27. The VHH antibody of any one of embodiments 1-23, which is selected from VHH antibody Bm7E11 having a VHH sequence as shown in SEQ. ID NO: 17 or a VHH antibody, which is a variant thereof.
28. The VHH antibody of any one of embodiments 1-23, which is selected from VHH antibody Bm7F03 having a VHH sequence as shown in SEQ. ID NO: 21 or a VHH antibody, which is a variant thereof.
29. The VHH antibody of any one of embodiments 1-23, which is selected from VHH antibody Bm7G08 having a VHH sequence as shown in SEQ. ID NO: 25 or a VHH antibody, which is a variant thereof.
30. The VHH antibody of any one of embodiments 1-23, which is selected from VHH antibody Bm7D04 having a VHH sequence as shown in SEQ. ID NO: 29 or a VHH antibody, which is a variant thereof.
31. The VHH antibody of any one of embodiments 1-23, which is selected from VHH antibody Bm7H02 having a VHH sequence as shown in SEQ. ID NO: 33 or a VHH antibody, which is a variant thereof.
32. The VHH antibody of any one of embodiments 1-23, which is selected from VHH antibody Bm8D12 having a VHH sequence as shown in SEQ. ID NO: 37 or a VHH antibody, which is a variant thereof.
33. The VHH antibody of any one of embodiments 1-23, which is selected from VHH antibody Bm8B10 having a VHH sequence as shown in SEQ. ID NO: 41 or a VHH antibody, which is a variant thereof.
34. The VHH antibody of any one of embodiments 1-23, which is selected from VHH antibody Bm8D09 having a VHH sequence as shown in SEQ. ID NO: 45 or a VHH antibody, which is a variant thereof.
35. The VHH antibody of any one of embodiments 1-23, which is selected from VHH antibody Bm8H12 having a VHH sequence as shown in SEQ. ID NO: 49 or a VHH antibody, which is a variant thereof.
36. The VHH antibody of any one of embodiments 1-23, which is selected from VHH antibody Bm8C06 having a VHH sequence as shown in SEQ. ID NO: 53 or a VHH antibody, which is a variant thereof.
37. The VHH antibody of any one of embodiments 1-23, which is selected from VHH antibody Bm8H07 having a VHH sequence as shown in SEQ. ID NO: 57 or a VHH antibody, which is a variant thereof.
38. The VHH antibody of any one of embodiments 1-23, which is selected from VHH antibody Bm8G09 having a VHH sequence as shown in SEQ. ID NO: 61 or a VHH antibody, which is a variant thereof.
39. The VHH antibody of any one of embodiments 1-23, which is selected from VHH antibody Bm8G10 having a VHH sequence as shown in SEQ. ID NO: 65 or a VHH antibody, which is a variant thereof.
40. The VHH antibody of any one of embodiments 1-23, which is selected from VHH antibody Bm8F05 having a VHH sequence as shown in SEQ. ID NO: 69 or a VHH antibody, which is a variant thereof.
41. The VHH antibody of any one of embodiments 1-23, which is selected from VHH antibody Bm8H10 having a VHH sequence as shown in SEQ. ID NO: 73 or a VHH antibody, which is a variant thereof.
42. The VHH antibody of any one of embodiments 1-23, which is selected from VHH antibody Bm8G06 having a VHH sequence as shown in SEQ. ID NO: 77 or a VHH antibody, which is a variant thereof.
43. The VHH antibody of any one of embodiments 1-23, which is selected from VHH antibody Bm8G07 having a VHH sequence as shown in SEQ. ID NO: 81 or a VHH antibody, which is a variant thereof.
44. The VHH antibody of any one of embodiments 1-23, which is selected from VHH antibody Bm8G12 having a VHH sequence as shown in SEQ. ID NO: 85 or a VHH antibody, which is a variant thereof.
45. The VHH antibody of any one of embodiments 1-23, which is selected from VHH antibody Bm27C04 having a VHH sequence as shown in SEQ. ID NO: 89 or a VHH antibody, which is a variant thereof.
46. The VHH antibody of any one of embodiments 1-23, which is selected from VHH antibody Bm27C06 having a VHH sequence as shown in SEQ. ID NO: 93 or a VHH antibody, which is a variant thereof.
47. The VHH antibody of any one of embodiments 1-23, which is selected from VHH antibody Bm27E06 having a VHH sequence as shown in SEQ. ID NO: 97 or a VHH antibody, which is a variant thereof.
48. The VHH antibody of any one of embodiments 1-23, which is selected from VHH antibody Bm27H03 having a VHH sequence as shown in SEQ. ID NO: 101 or a VHH antibody, which is a variant thereof.
49. The VHH antibody of any one of embodiments 1-23, which is selected from VHH antibody Bm27C11 having a VHH sequence as shown in SEQ. ID NO: 105 or a VHH antibody, which is a variant thereof.
50. The VHH antibody of any one of embodiments 1-23, which is selected from VHH antibody Bm27D06 having a VHH sequence as shown in SEQ. ID NO: 109 or a VHH antibody, which is a variant thereof.
51. The VHH antibody of any one of the preceding embodiments, which is non-glycosylated, or which is glycosylated.
52. The VHH antibody of any one of the preceding embodiments, which is produced in a bacterium, e.g., *E. coli,* or in a yeast, e.g., *Pichia pastoris,* or in a human or animal cell, e.g., an insect cell or a mammalian cell, e.g., a CHO cell.
53. The VHH antibody of any one of the preceding embodiments, which is in a monovalent format.
54. The VHH antibody of any one of embodiments 1-52, which is in a multimeric format.
55. The VHH antibody of embodiment 54, which is in a dimeric format.
56. The VHH antibody of embodiment 55, which is in a homodimeric or in a heterodimeric format.
57. The VHH antibody of any one of the preceding embodiments, which is covalently or non-covalently conjugated to a heterologous moiety, e.g., a labeling group, a capture group, or an effector group, wherein the heterologous moiety is particularly selected from a fluorescence group, biotin, an enzyme such as a peroxidase, phosphatase or luciferase, a hapten, an affinity tag, or a nucleic acid such as an oligonucleotide.
58. The VHH antibody of any one of the preceding embodiments, which is fused to a heterologous polypeptide moiety, including an appropriate Fc fragment, serum albumin or an albumin-binding moiety.
59. The VHH antibody of any one of the preceding embodiments, which is conjugated to one or several non-peptidic polymer moieties, preferably hydrophilic polymer moieties, such as polyethylene glycol (PEG).
60.A hetero-multimeric VHH antibody comprising two or more different VHH antibody units, e.g., 2, 3 or 4 different VHH antibody units wherein at least one VHH antibody unit comprises a VHH antibody of any of embodiments 1-52.
61. The hetero-multimeric VHH antibody of embodiment 60, which comprises at least two VHH antibody units recognizing basigin, particularly different epitopes on basigin.
62. The hetero-multimeric VHH antibody of embodiment 60 or 61, which comprises at least two VHH antibody units selected from different VHH antibody classes (i), (ii), (iii), (iv), (v), (vi), (vii) and (viii) as follows:
   (i) a VHH antibody, which binds to the same or an overlapping epitope on basigin as the VHH antibody Bm7D12 having a VHH sequence as shown in SEQ. ID NO: 5;
   (ii) a VHH antibody, which binds to the same or an overlapping epitope on basigin as the VHH antibody Bm8D12 having a VHH sequence as shown in SEQ. ID NO: 37;
   (iii) a VHH antibody, which binds to the same or an overlapping epitope on basigin as the VHH antibody Bm8G10 having a VHH sequence as shown in SEQ. ID NO: 65;
   (iv) a VHH antibody, which binds to the same or an overlapping epitope on basigin as the VHH antibody Bm8H10 having a VHH sequence as shown in SEQ. ID NO: 73;
   (v) a VHH antibody, which competes with the VHH antibody Bm8G07 having a VHH sequence as shown in SEQ. ID NO: 81 for the binding to basigin;
   (vi) a VHH antibody, which binds to the same or an overlapping epitope on basigin as the VHH antibody Bm8G12 having a VHH sequence as shown in SEQ. ID NO: 85;
   (vii) a VHH antibody, which binds to the same or an overlapping epitope on basigin as the VHH antibody Bm27E06 having a VHH sequence as shown in SEQ. ID NO: 97; and
   (viii) a VHH antibody, which binds to the same or an overlapping epitope on basigin as with the VHH antibody Bm27C11 having a VHH sequence as shown in SEQ. ID NO: 105.
63.A set of two or more different VHH antibodies recognizing basigin, comprising at least one VHH antibody of any of embodiments 1-62, particularly at least one VHH antibody in a monovalent format.
64. The set of embodiment 63 comprising at least two VHH antibodies selected from at least two different VHH classes (i), (ii), (iii), (iv), (v), (vi), (vii) and (viii) as follows:
   (i) a VHH antibody, which binds to the same or an overlapping epitope on basigin as the VHH antibody Bm7D12 having a VHH sequence as shown in SEQ. ID NO: 5;
   (ii) a VHH antibody, which binds to the same or an overlapping epitope on basigin as the VHH antibody Bm8D12 having a VHH sequence as shown in SEQ. ID NO: 37;
   (iii) a VHH antibody, which binds to the same or an overlapping epitope on basigin as the VHH antibody Bm8G10 having a VHH sequence as shown in SEQ. ID NO: 65;
   (iv) a VHH antibody, which binds to the same or an overlapping epitope on basigin as the VHH antibody Bm8H10 having a VHH sequence as shown in SEQ. ID NO: 73;
   (v) a VHH antibody, which competes with the VHH antibody Bm8G07 having a VHH sequence as shown in SEQ. ID NO: 81 for the binding to basigin;
   (vi) a VHH antibody, which binds to the same or an overlapping epitope on basigin as the VHH antibody Bm8G12 having a VHH sequence as shown in SEQ. ID NO: 85;
   (vii) a VHH antibody, which binds to the same or an overlapping epitope on basigin as the VHH antibody Bm27E06 having a VHH sequence as shown in SEQ. ID NO: 97; and
   (viii) a VHH antibody, which binds to the same or an overlapping epitope on basigin as with the VHH antibody Bm27C11 having a VHH sequence as shown in SEQ. ID NO: 105.
65. The VHH antibody of any one of embodiments 1-62 or the set of any one of embodiments 63-64 for use as a medicament, particularly in human medicine.
66. The VHH antibody of any one of embodiments 1-62 or the set of any one of embodiments 63-64 for use in the prevention, treatment and/or mitigation of a condition caused by, associated with and/or accompanied by an activity of basigin.
67. The VHH antibody of any one of embodiments 1-62 or the set of any one of embodiments 63-64 for the use of embodiment 66 wherein the activity of basigin is related to its receptor function, particularly to its receptor function on erythrocytes.
68. The VHH antibody of any one of embodiments 1-62 or the set of any one of embodiments 63-64 for use in the prevention, treatment and/or mitigation of a condition caused by, associated with and/or accompanied by an infection with a pathogen that invades mammalian cells by interaction with basigin.
69. The VHH antibody of any one of embodiments 1-62 or the set of any one of embodiments 63-64 for the use of embodiment 68 wherein the pathogen is a bacterium including but not limited to *Listeria monocytogenes* and *Neisseria meningitidis*, a virus, or a parasite including but not limited to *Plasmodium falciparum.*
70. The VHH antibody of any one of embodiments 1-62 or the set of any one of embodiments 63-64 for the use of embodiment 69 wherein the condition is malaria.
71. The VHH antibody of any one of embodiments 1-62 or the set of any one of embodiments 63-64 for use in the prevention, treatment and/or mitigation of a cancer caused by, associated with and/or accompanied by an activity of basigin including but not limited to induction of matrix metalloproteinases, tumor cell invasion, angiogenesis, and/or interaction of basigin with monocarboxylate transporters (MCTs).
72. The VHH antibody of any one of embodiments 1-62 or the set of any one of embodiments 63-64 for use in the prevention, treatment and/or mitigation of a cardiovascular disorder including but not limited to atherosclerosis and myocardial infarction.
73. The VHH antibody of any one of embodiments 1-62 or the set of any one of any or the set of any one of embodiments 63-64 for the use of any one of embodiments 65-72 in a human subject.
74. The VHH antibody of any one of embodiments 1-62 or the set of any one of embodiments 63-64 for the use of any one of embodiments 65-73, which is administered locally.
75. The VHH antibody of any one of embodiments 1-62 or the set of any one of embodiments 63-64 for the use of any one of embodiments 65-73, which is administered systemically.
76. The VHH antibody of any one of embodiments 1-62 or the set of any one of embodiments 63-64 for the use of any one of embodiments 65-75, which is administered topically, parenterally, e.g., intravenously, intramuscularly or subcutaneously, pulmonary, e.g., by inhalation, or nasally, e.g., by spraying.
77. The VHH antibody of any one of embodiments 1-62 or the set of any one of embodiments 63-64 for the use of any one of embodiments 65-76, which is administered as a monotherapy.
78. The VHH antibody of any one of embodiments 1-62 or the set of any one of embodiments 63-64 for the use of any one of embodiments 65-76, which is administered sequentially and/or simultaneously as a combination therapy with at least one further active agent.
79. The VHH antibody of any one of embodiments 1-62 or the set of any one of embodiments 63-64 for the use of embodiment 78, which is administered in combination with an anti-malaria agent such as atovaquone, and/or pyrimethamine.
80. A pharmaceutical composition comprising as an active agent the VHH antibody of any one of embodiments 1-62 or the set of any one of embodiments 63-64, and a pharmaceutically acceptable carrier.
81.A nucleic acid molecule encoding a VHH antibody or a VHH antibody containing unit according to any one of embodiments 1-62, preferably in operative linkage with a heterologous expression control sequence.
82. A vector comprising a nucleic acid molecule according to embodiment 81.
83. A recombinant cell or non-human organism transformed or transfected with a nucleic acid molecule according to embodiment 81 or a vector according to embodiment 82.
84. The cell or organism of embodiment 83, which is selected from a bacterium such as E. *coli Bacillus sp.*, a unicellular eukaryotic organism, e.g., yeast such as *Pichia pastoris*, or *Leishmania*, an insect cell, a mammalian cell, or a plant cell.
85.A method for recombinant production of a VHH antibody of any one of embodiments 1-62, comprising cultivating a cell or an organism of embodiment 83 or 84 in a suitable medium and obtaining the VHH antibody from the cell or organism or from the medium.
86. The method of embodiment 85, comprising cultivating a yeast such as *Pichia pastoris* and obtaining the VHH antibody from the medium.
87. Use of the VHH antibody of any one of embodiments 1-62 or the set of any one of embodiments 63-64 for detecting basigin in a sample.
88. The use of embodiment 87, wherein the sample is a biological sample, e.g., a body fluid such as saliva, sputum, a lung lavage, a swab, blood, serum or plasma, a stool sample, a tissue sample, or a biopsy sample.
89. The use of embodiment 87 or 88, wherein the detection comprises the binding of at least 2 different VHH antibodies to a basigin molecule.
90. The use of embodiment 89, wherein the detection comprises the binding of at least 2 different VHH antibodies, e.g., 2, 3, or 4 VHH antibodies each directed against a different epitope, to a basigin molecule.
91.The use of any one of embodiments 87-90, wherein the detection comprises a sandwich assay, e.g., a sandwich ELISA.
92.A method for the prevention, treatment and/or mitigation of a condition caused by, associated with and/or accompanied by an activity of basigin comprising administering an effective dose of the VHH antibody of any one of embodiments 1-62 or the set of any one of embodiments 63-64 or the pharmaceutical composition of embodiment 80 to a subject in need thereof, particularly to a human subject.

### Description of the Invention

### VHH antibodies

The present invention relates to a VHH antibody recognizing basigin.

The VHH antibody may be a monovalent heavy chain-only antibody comprising a CDR1 domain, a CDR2 domain and a CDR3 domain linked by framework regions including, but not being limited to, whole VHH antibodies, e.g., native VHH antibodies comprising framework regions derived from camelids, and modified VHH antibodies comprising modified framework regions, VHH antibody fragments and VHH antibody fusion proteins, e.g. a fusion protein with an immunoglobulin or non-immunoglobulin peptide or polypeptide, as long as it shows the properties according to the invention.

The VHH antibody of the present invention may be glycosylated or non-glycosylated.

There are several methods known in the art for determining the CDR sequences of a given antibody molecule, but there is no standard unequivocal method. Determination of CDR sequences from antibody heavy chain variable regions can be made according to any method known in the art, including, but not limited to, the methods known as Kabat, Chothia, and IMGT. A selected set of CDRs may include sequences identified by more than one method, namely, some CDR sequences may be determined using Kabat and some using IMGT, for example. According to some embodiments of the present invention, the CDR sequences of a VHH variable region are determined using the Kabat method. CDRs may also be defined through a multiple alignment (with many other VHH antibodies), to identify the hot-spots of variability and relate them to a standard VHH antibody structure. It is also possible to define CDRs by analyzing the structure of the VHH antibody and deciding what is a loop and what is the antibody's scaffold. In some cases, CDR-adjacent residues are also variable, and are therefore included in the CDR definition.

The present invention is also directed to a covalent or non-covalent conjugate of a VHH antibody molecule to a non-proteinaceous structure, for example, a labeling group, a capture group such a solid phase-binding group, or an effector group such as a toxin. For example, the heterologous moiety may be from a fluorescence group, biotin, an enzyme such as a peroxidase, phosphatase, or luciferase, a hapten, an affinity tag, or a nucleic acid such as an oligonucleotide.

The VHH antibody of the present invention is particularly a monoclonal VHH antibody characterized by a specific amino acid sequence. The VHH antibody may be produced in a prokaryotic host cell, a yeast cell or a mammalian cell, e.g., a CHO cell. In certain embodiments, the VHH antibody is non-glycosylated. In certain embodiments, the VHH antibody is glycosylated, wherein a carbohydrate structure may be derived from a glycosylation site introduced into the VHH sequence and/or from a fusion partner.

A VHH antibody according to the present invention is characterized by (i) a CDR3 sequence, (ii) a combination of a CDR1 sequence, a CDR2 sequence, and a CDR3 sequence, (iii) by a complete VHH sequence, or (iv) by competition with a specific reference antibody. Specific CDR and VHH sequences are provided in the Tables, Figures and the Sequence Listing.

According to the present invention, sequences related to the above sequences are encompassed. These related sequences are defined by having a minimum identity to a specifically indicated amino acid sequence, e.g., a CDR or VHH sequence. This identity is indicated over the whole length of the respective reference sequence and may be determined by using well-known algorithms such as BLAST.

In particular embodiments, a related CDR3 sequence has an identity of at least 80% or at least 90% or at least 95% to a specifically indicated CDR3 sequence, e.g., a substitution of 1, 2, or 3 amino acids.

In particular embodiments, a related combination of a CDR1 sequence, a CDR2 sequence, and a CDR3 sequence has an identity of at least 80% or at least 90% or at least 95% to a specifically indicated combination of a CDR1 sequence, a CDR2 sequence, and a CDR3 sequence, e.g., a substitution of 1, 2, 3, 4, 5 or 6 amino acids by different amino acids.

In particular embodiments, a related VHH sequence has an identity of least 70%, at least 80%, at least 90%, at least 95% or at least 99% to a VHH sequence, e.g., a substitution of 1, 2, 3, 4, 5 or up to 20 amino acids.

Further, the invention refers to a VHH antibody, which binds to the same or an overlapping epitope on basigin as a specific VHH antibody disclosed herein for the binding to basigin. For example, the invention refers to a VHH antibody, which binds to the same or an overlapping epitope on basigin as a reference antibody, e.g., VHH antibody Bm7D12, Bm8D12, Bm8G10, Bm8H10, Bm8G06, or Bm27E06 (binding to epitope 1 on basigin), with VHH antibody Bm8G07, or Bm8G12 (binding to epitope 2 on basigin), or VHH antibody Bm27C11 (binding to epitope 3 on basigin). Binding to the same or an overlapping epitope may be determined by label-free biolayer interferometry performed as a cross-competition or epitope binning assay using a label-free detection system, e.g., an Octet^{®} system from Sartorius, according to the manufacturer's instructions.

In certain embodiments, the VHH antibody interacts with the same amino acids on basigin as the reference antibody Bm7D12, particularly a VHH antibody which interacts with amino acid residues F27, T28, T29, V30, E31, D32, G34, S35, I37, S78, Q81, Q100, L101, H102, G103, P104, R106, and E129 of basigin in the numbering of basigin isoform 2 (UniProt P35613-2).

In certain embodiments, the VHH antibody interacts with the same amino acids on basigin as the reference antibody Bm8D12, particularly a VHH antibody which interacts with amino acid residues T25, V26, F27, T28, T29, V30, E31, L33, G34, L38, T40, S42, N44, V131, P132, P133, Q164, S190, and K191 of basigin in the numbering of basigin isoform 2 (UniProt P35613-2).

In particular embodiments, at least one amino acid of a reference sequence, including an amino acid in a CDR1, CDR2 or CDR3 sequence and/or an amino acid in a framework region, is replaced by another amino acid, while preserving structural integrity and epitope-binding of the VHH antibody. These exchanges can be conservative (i.e., by a similar amino acid) or non-conservative.

In further particular embodiments, at least one amino acid of a reference sequence, including an amino acid in a CDR1, CDR2 or CDR3 sequence and/or an amino acid in a framework region, is replaced by a conservative amino acid substitution, i.e. a substitution of an amino acid by another amino acid with similar biochemical properties, for example a substitution of an aliphatic amino acid, e.g. Gly, Ala, Val, Leu, or Ile, for another aliphatic amino acid; a substitution of a basic amino acid, e.g. His, Lys orArg, against another basic amino acid or against Met; a substitution of an acidic amino acid or an amide thereof, e.g., Asp, Glu, Asn or Gln, against another acidic amino acid or an amide thereof; a substitution of an aromatic amino acid, e.g., Phe, Tyr or Trp, against another aromatic amino acid.

In further particular embodiments, the VHH antibody is selected from antibody Bm7D12 having a VHH sequence as shown in SEQ. ID NO: 5 or a VHH antibody, which is a variant thereof. In certain embodiments, 1, 2, 3, 4, 5, 6, 7, 8, 9 or 10 amino acids of SEQ. ID NO: 5 are replaced by another amino acid.

In further particular embodiments, the VHH antibody is selected from antibody Bm7H08 having a VHH sequence as shown in SEQ. ID NO: 9 or a VHH antibody, which is a variant thereof. In certain embodiments, 1, 2, 3, 4, 5, 6, 7, 8, 9 or 10 amino acids of SEQ. ID NO: 9 are replaced by another amino acid.

In further particular embodiments, the VHH antibody is selected from antibody Bm7G12 having a VHH sequence as shown in SEQ. ID NO: 13 or a VHH antibody, which is a variant thereof. In certain embodiments, 1, 2, 3, 4, 5, 6, 7, 8, 9 or 10 amino acids of SEQ. ID NO: 13 are replaced by another amino acid.

In further particular embodiments, the VHH antibody is selected from antibody Bm7E11 having a VHH sequence as shown in SEQ. ID NO: 17 or a VHH antibody, which is a variant thereof. In certain embodiments, 1, 2, 3, 4, 5, 6, 7, 8, 9 or 10 amino acids of SEQ. ID NO: 17 are replaced by another amino acid.

In further particular embodiments, the VHH antibody is selected from antibody Bm7F03 having a VHH sequence as shown in SEQ. ID NO: 21 or a VHH antibody, which is a variant thereof. In certain embodiments, 1, 2, 3, 4, 5, 6, 7, 8, 9 or 10 amino acids of SEQ. ID NO: 21 are replaced by another amino acid.

In further particular embodiments, the VHH antibody is selected from antibody Bm7G08 having a VHH sequence as shown in SEQ. ID NO: 25 or a VHH antibody, which is a variant thereof. In certain embodiments, 1, 2, 3, 4, 5, 6, 7, 8, 9 or 10 amino acids of SEQ. ID NO: 25 are replaced by another amino acid.

In further particular embodiments, the VHH antibody is selected from antibody Bm7D04 having a VHH sequence as shown in SEQ. ID NO: 29 or a VHH antibody, which is a variant thereof. In certain embodiments, 1, 2, 3, 4, 5, 6, 7, 8, 9 or 10 amino acids of SEQ. ID NO: 29 are replaced by another amino acid.

In further particular embodiments, the VHH antibody is selected from antibody Bm7H02 having a VHH sequence as shown in SEQ. ID NO: 33 or a VHH antibody, which is a variant thereof. In certain embodiments, 1, 2, 3, 4, 5, 6, 7, 8, 9 or 10 amino acids of SEQ. ID NO: 33 are replaced by another amino acid.

In further particular embodiments, the VHH antibody is selected from antibody Bm8D12 having a VHH sequence as shown in SEQ. ID NO: 37 or a VHH antibody, which is a variant thereof. In certain embodiments, 1, 2, 3, 4, 5, 6, 7, 8, 9 or 10 amino acids of SEQ. ID NO: 37 are replaced by another amino acid.

In further particular embodiments, the VHH antibody is selected from antibody Bm8B10 having a VHH sequence as shown in SEQ. ID NO: 41 or a VHH antibody, which is a variant thereof. In certain embodiments, 1, 2, 3, 4, 5, 6, 7, 8, 9 or 10 amino acids of SEQ. ID NO: 41 are replaced by another amino acid.

In further particular embodiments, the VHH antibody is selected from antibody Bm8D09 having a VHH sequence as shown in SEQ. ID NO: 45 or a VHH antibody, which is a variant thereof. In certain embodiments, 1, 2, 3, 4, 5, 6, 7, 8, 9 or 10 amino acids of SEQ. ID NO: 45 are replaced by another amino acid.

In further particular embodiments, the VHH antibody is selected from antibody Bm8H12 having a VHH sequence as shown in SEQ. ID NO: 49 or a VHH antibody, which is a variant thereof. In certain embodiments, 1, 2, 3, 4, 5, 6, 7, 8, 9 or 10 amino acids of SEQ. ID NO: 49 are replaced by another amino acid.

In further particular embodiments, the VHH antibody is selected from antibody Bm8C06 having a VHH sequence as shown in SEQ. ID NO: 53 or a VHH antibody, which is a variant thereof. In certain embodiments, 1, 2, 3, 4, 5, 6, 7, 8, 9 or 10 amino acids of SEQ. ID NO: 53 are replaced by another amino acid.

In further particular embodiments, the VHH antibody is selected from antibody Bm8H07 having a VHH sequence as shown in SEQ. ID NO: 57 or a VHH antibody, which is a variant thereof. In certain embodiments, 1, 2, 3, 4, 5, 6, 7, 8, 9 or 10 amino acids of SEQ. ID NO: 57 are replaced by another amino acid.

In further particular embodiments, the VHH antibody is selected from antibody Bm8G09 having a VHH sequence as shown in SEQ. ID NO: 61 or a VHH antibody, which is a variant thereof. In certain embodiments, 1, 2, 3, 4, 5, 6, 7, 8, 9 or 10 amino acids of SEQ. ID NO: 61 are replaced by another amino acid.

In further particular embodiments, the VHH antibody is selected from antibody Bm8G10 having a VHH sequence as shown in SEQ. ID NO: 65 or a VHH antibody, which is a variant thereof. In certain embodiments, 1, 2, 3, 4, 5, 6, 7, 8, 9 or 10 amino acids of SEQ. ID NO: 65 are replaced by another amino acid.

In further particular embodiments, the VHH antibody is selected from antibody Bm8F05 having a VHH sequence as shown in SEQ. ID NO: 69 or a VHH antibody, which is a variant thereof. In certain embodiments, 1, 2, 3, 4, 5, 6, 7, 8, 9 or 10 amino acids of SEQ. ID NO: 69 are replaced by another amino acid.

In further particular embodiments, the VHH antibody is selected from antibody Bm8H10 having a VHH sequence as shown in SEQ. ID NO: 73 or a VHH antibody, which is a variant thereof. In certain embodiments, 1, 2, 3, 4, 5, 6, 7, 8, 9 or 10 amino acids of SEQ. ID NO: 73 are replaced by another amino acid.

In further particular embodiments, the VHH antibody is selected from antibody Bm8G06 having a VHH sequence as shown in SEQ. ID NO: 77 or a VHH antibody, which is a variant thereof. In certain embodiments, 1, 2, 3, 4, 5, 6, 7, 8, 9 or 10 amino acids of SEQ. ID NO: 77 are replaced by another amino acid.

In further particular embodiments, the VHH antibody is selected from antibody Bm8G07 having a VHH sequence as shown in SEQ. ID NO: 81 or a VHH antibody, which is a variant thereof. In certain embodiments, 1, 2, 3, 4, 5, 6, 7, 8, 9 or 10 amino acids of SEQ. ID NO: 81 are replaced by another amino acid.

In further particular embodiments, the VHH antibody is selected from antibody Bm8G12 having a VHH sequence as shown in SEQ. ID NO: 85 or a VHH antibody, which is a variant thereof. In certain embodiments, 1, 2, 3, 4, 5, 6, 7, 8, 9 or 10 amino acids of SEQ. ID NO: 85 are replaced by another amino acid.

In further particular embodiments, the VHH antibody is selected from antibody Bm27C04 having a VHH sequence as shown in SEQ. ID NO: 89 or a VHH antibody, which is a variant thereof. In certain embodiments, 1, 2, 3, 4, 5, 6, 7, 8, 9 or 10 amino acids of SEQ. ID NO: 89 are replaced by another amino acid.

In further particular embodiments, the VHH antibody is selected from antibody Bm27C06 having a VHH sequence as shown in SEQ. ID NO: 93 or a VHH antibody, which is a variant thereof. In certain embodiments, 1, 2, 3, 4, 5, 6, 7, 8, 9 or 10 amino acids of SEQ. ID NO: 93 are replaced by another amino acid.

In further particular embodiments, the VHH antibody is selected from antibody Bm27E06 having a VHH sequence as shown in SEQ. ID NO: 97 or a VHH antibody, which is a variant thereof. In certain embodiments, 1, 2, 3, 4, 5, 6, 7, 8, 9 or 10 amino acids of SEQ. ID NO: 97 are replaced by another amino acid.

In further particular embodiments, the VHH antibody is selected from antibody Bm27H03 having a VHH sequence as shown in SEQ. ID NO: 101 or a VHH antibody, which is a variant thereof. In certain embodiments, 1, 2, 3, 4, 5, 6, 7, 8, 9 or 10 amino acids of SEQ. ID NO: 101 are replaced by another amino acid.

In further particular embodiments, the VHH antibody is selected from antibody Bm27C11 having a VHH sequence as shown in SEQ. ID NO: 105 or a VHH antibody, which is a variant thereof. In certain embodiments, 1, 2, 3, 4, 5, 6, 7, 8, 9 or 10 amino acids of SEQ. ID NO: 105 are replaced by another amino acid.

In further particular embodiments, the VHH antibody is selected from antibody Bm27D06 having a VHH sequence as shown in SEQ. ID NO: 109 or a VHH antibody, which is a variant thereof. In certain embodiments, 1, 2, 3, 4, 5, 6, 7, 8, 9 or 10 amino acids of SEQ. ID NO: 109 are replaced by another amino acid.

Further, the present invention relates to a nucleic acid molecule, e.g., a DNA molecule, encoding a VHH as indicated above, or a vector, comprising said nucleic acid molecule as indicated above in operative linkage with an expression control sequence, particularly with a heterologous expression control sequence. Furthermore, the invention relates to a cell comprising a nucleic acid molecule or a vector as described above. Vectors for the recombinant production of VHH antibodies are well-known in the art. In certain embodiments, the vector is an extrachromosomal vector. In other embodiments, the vector is a vector for genomic integration. The cell may be a known host cell for producing antibodies or antibody fragments, e.g., a prokaryotic cell such as an *E*. *coli* or a *Bacillus sp.* cell, a yeast cell, particularly a *Pichia* yeast cell, an insect cell, e.g., an Sf-9 cell, or a mammalian cell, e.g., a CHO cell, or a plant cell. In certain embodiments, the cell comprises the nucleic acid or the vector extrachromosomally. In other embodiments, the cell comprises the nucleic acid or the vector integrated into the genome, e.g., as a genomically integrated expression cassette.

Still a further aspect of the present invention is a method of recombinantly producing a VHH antibody by growing a cell as described above in a culture medium and obtaining the VHH antibody from the cell or the culture medium. Suitable culture media and culture conditions are well known in the art.

### Binding to Basigin

The VHH antibodies of the present invention bind to basigin. The inventors have identified VHH antibodies, which bind with high affinity to different epitopes on basigin, as shown in Table 1, supra.

In the context of the present disclosure the term "basigin" encompasses basigin from different organisms, particularly from different mammalian organisms including human basigin. In particular, the term "basigin" encompasses basigin isoform 2 ectodomain (UniProt P35613-2, residues 24-204) (SEQ ID NO: 1).

It should be noted, however, that the term "basigin" also encompasses naturally occurring variants of basigin and genetically modified constructs as described herein.

The inventors have performed their Octet assays with a basigin variant that contained two ectopic cysteines (as shown in SEQ. ID NO: 2) and that was biotinylated through maleimide chemistry at cysteines and determined x-ray crystals structures with basigin shown in SEQ ID NO: 3.

In certain embodiments, the VHH antibody of the present invention binds to basigin wherein the binding affinity expressed as dissociation constant K_{D} is about 500 pM or less, about 100 pM or less, or about 10 pM or less. The binding affinity may be determined as described herein in detail in the Examples using the polypeptides of SEQ. ID NO: 1, 2 and 3 as described above.

### Inhibition of PfRH5 binding to basigin

The VHH antibodies of the present invention are capable of inhibiting the binding of PfRH5 to basigin of human erythrocytes, as shown in Table 1, supra.

This particularly applies to VHH antibodies recognizing epitope 1 of basigin as shown in Table 1, supra.

In certain embodiments, the VHH antibody shields the PfRH5-binding site of basigin. In further embodiments, the VHH antibody 'freezes' the linker between the two basigin Ig domains in a conformation that is incompatible with PfRH5 binding. In still further embodiments, the VHH antibody shields the PfRH5-binding site of basigin and freezes the linker between the two basigin Ig domains in a conformation that is incompatible with PfRH5 binding.

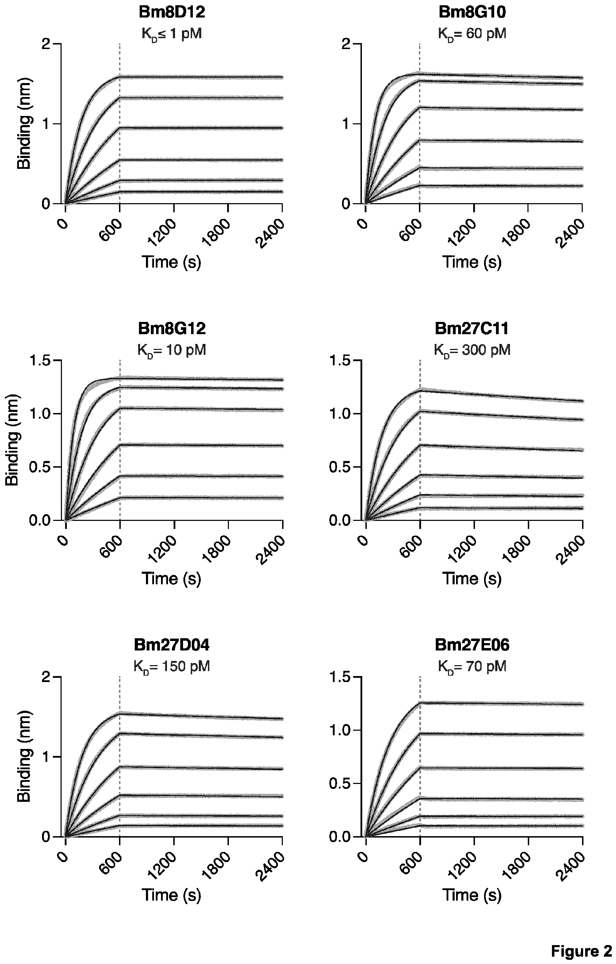

In certain embodiments, the VHH antibody of the present invention inhibits an entry of Plasmodia into erythrocytes, particularly at a concentration that is equivalent to the amount of available basigin molecules on erythrocytes. Thus, the effective nanobody concentration is not limited by the affinity to basigin (which is actually in the picomolar range, as shown in Figure 2, supra), but by stoichiometry, i.e., by the need to cover the majority of basigin molecules present on erythrocytes.

### Stability

For the intended therapeutic application, the anti-basigin antibodies should not only be highly potent in basigin binding, but they should also be developable as biological drugs. This includes that they are stable enough to survive a lengthy, large-scale production process as well as transportation and storage (ideally for years in liquid formulation) without aggregation or loss of activity.

A good predictor for stability is thermostability, which can be measured, e.g., by thermal shift assays or specifically by differential scanning fluorimetry. The present inventors have identified several thermostable or hyperthermostable VHH antibodies as shown in Table 1, supra.

In a particular embodiment, the invention relates to a VHH antibody, which is stable, particularly thermostable, or hyperthermostable. Preferably, the VHH antibody has a melting point (melting temperature) of at least about 60°C, of at least 80°C or of at least about 95°C when measured under non-reducing conditions, and/or a melting point (melting temperature) of at least about 30°C, of at least about 50°C or of at least about 95°C when measured under reducing conditions. Melting temperatures are determined as described herein.

### Sets of VHH antibodies

In a further aspect, the present invention relates to a set comprising at least 2, 3, 4 or more of the above VHH antibodies. In such a set, the individual VHH antibodies are present in suitable molar ratios. Typically, the molar ratios are in the range of about 2:1 to about 1:2, particularly about 1.5:1 to about 1:1.5, even more particularly about 1:1. In certain embodiments, the VHH antibody set may comprise a single composition wherein the VHH antibodies in said set consist of a predetermined number of different species of VHH antibodies as described above. The VHH antibody set may comprise a plurality of compositions each comprising a different species of VHH antibody as described above. The set of the present invention may be free from other VHH antibodies.

In particular embodiments, the set of VHH antibodies comprises at least two, e.g., two or three complementary VHH antibodies, i.e., VHH antibodies directed against different epitopes of basigin, e.g., a VHH antibody directed against epitope 1, a VHH antibody directed against epitope 2, a VHH antibody directed against epitope 3 as defined herein. More particularly, a set of VHH antibodies comprises at least one VHH antibody which recognizes epitope 1 of basigin.

For example, the set of VHH antibodies comprises at least two, e.g., two or three complementary VHH antibodies, selected from at least two or three different VHH antibody classes (i), (ii), (iii), (iv), (v), (vi), (vii) and (viii) and more particularly from at least two or three different epitope 1-binding VHH antibody classes (i), (ii), (iii), (iv) and (vii) as follows:
(i) a VHH antibody, which binds to the same or an overlapping epitope on basigin as the VHH antibody Bm7D12 having a VHH sequence as shown in SEQ. ID NO: 5;
(ii) a VHH antibody, which binds to the same or an overlapping epitope on basigin as the VHH antibody Bm8D12 having a VHH sequence as shown in SEQ. ID NO: 37;
(iii) a VHH antibody, which binds to the same or an overlapping epitope on basigin as the VHH antibody Bm8G10 having a VHH sequence as shown in SEQ. ID NO: 65;
(iv) a VHH antibody, which binds to the same or an overlapping epitope on basigin as the VHH antibody Bm8H10 having a VHH sequence as shown in SEQ. ID NO: 73;
(v) a VHH antibody, which competes with the VHH antibody Bm8G07 having a VHH sequence as shown in SEQ. ID NO: 81 for the binding to basigin;
(vi) a VHH antibody, which binds to the same or an overlapping epitope on basigin as the VHH antibody Bm8G12 having a VHH sequence as shown in SEQ. ID NO: 85;
(vii) a VHH antibody, which binds to the same or an overlapping epitope on basigin as the VHH antibody Bm27E06 having a VHH sequence as shown in SEQ. ID NO: 97; and
(viii) a VHH antibody, which binds to the same or an overlapping epitope on basigin as with the VHH antibody Bm27C11 having a VHH sequence as shown in SEQ. ID NO: 105.

A particularly preferred set of VHH antibodies includes at least two different protective VHH antibodies, e.g., VHH antibodies recognizing epitope 1 of basigin. This is useful in case a patient develops an immune response against a therapeutic VHH antibody when treated multiple times. In certain embodiments, the set comprises at least two VHH antibodies each selected from selected from a different VHH antibody class (i), (ii), (iii), (iv), (v), (vi), (vii) and (viii), and particularly from a different epitope-1 binding VHH antibody class (i), (ii), (iii), (iv) and (vii), as herein defined.

Sets of different VHH antibodies are useful for therapeutic and diagnostic applications as described herein in detail below.

### Monovalent and multivalent VHH antibodies

In certain embodiments, the VHH antibody of the present invention is in a monovalent format, i.e., it has a single binding site for basigin. In these embodiments, the VHH antibody may be present as such or covalently or non-covalently attached to a heterologous moiety, e.g., a peptidic or non-peptidic moiety.

In further embodiments, the VHH antibody of the present invention is in in a multimeric, e.g., dimeric, trimeric or tetrameric format. In these embodiments, several VHH antibody units may be covalently or non-covalently attached to each other together via a linker and/or a multimerization, e.g., dimerization, trimerization or tetramerization moiety. In these embodiments, the VHH antibody of the present invention may be a homo-multimeric or hetero-multimeric antibody.

A hetero-multimeric VHH antibody comprises of two or more, e.g., 2, or 3 different VHH antibody units. The different VHH antibody units may be all directed against basigin and particularly against different epitopes (e.g., as defined herein) on basigin. More particularly, a hetero-multimeric VHH antibody comprises at least one VHH antibody unit which recognizes epitope 1 of basigin.

In certain embodiments, the hetero-multimeric VHH antibody comprises at least two VHH antibody units directed against basigin selected from VHH antibody classes (i), (ii), (iii), (iv), (v), (vi), (vii) and (viii), as herein defined above.

In certain embodiments, the hetero-multimeric VHH antibody comprises at least one VHH antibody unit directed against epitope 1 of basigin and at least one VHH antibody unit directed against epitope 2 basigin and/or and at least one VHH antibody unit directed against epitope 3 of basigin. Examples of specific VHH antibody fusions include a fusion comprising Bm7D12 and Bm8G12 and/or Bm27C11and a fusion comprising Bm8D12 and Bm8G12 and/or Bm27C11.

In certain embodiments, the VHH antibody may be covalently or non-covalently conjugated to a heterologous moiety, which is selected from a labeling group, a capture group, or an effector group, and wherein the heterologous moiety is particularly selected from a fluorescence group, biotin, an enzyme such as a peroxidase, phosphatase, or luciferase, a hapten, an affinity tag, or a nucleic acid such as an oligonucleotide. In further embodiments, the heterologous moiety is selected from human serum albumin, an albumin-binding moiety, a gamma globulin-binding moiety, a salvage receptor binding epitope, or an Fc fragment of an immunoglobulin molecule, e.g., IgA, IgD, IgE, IgG, IgM, or a subtype thereof. In certain embodiments, the Fc fragment is an appropriate Fc fragment suitable for therapeutic or diagnostic applications. In certain embodiments, the Fc fragment is devoid of effector function. In still further embodiments, the heterologous moiety is selected from one or several non-peptidic polymer moieties, preferably hydrophilic polymer moieties, such as polyethylene glycol (PEG).

In certain embodiments, the VHH antibody is a homodimeric VHH antibody, wherein a VHH antibody unit is covalently attached to a dimerization moiety, e.g., an immunoglobulin IgG Fc fragment.

In certain embodiments, the VHH antibody is a heterodimeric VHH antibody, particularly a covalently linked VHH heterodimer comprising a first VHH antibody and a second VHH antibody wherein the first VHH antibody and the second VHH antibody bind to different epitopes on basigin.

### Production of VHH antibodies

VHH antibodies including monomeric and multimeric VHH antibodies may be produced as described in WO 2022/023483 and WO 2022/023484, the contents of which are herein incorporated by reference, or by other methods well known in the art.

A VHH antibody may be recombinantly produced in a suitable host cell, e.g., in a prokaryotic or eukaryotic host cell or host organism. For this purpose, a nucleic acid molecule encoding the VHH antibody is introduced into the host cell or host organism and expressed in the host cell or host organism. The nucleic acid molecule may encode a monomeric VHH antibody or a subunit of a multimeric VHH antibody.

In a particular embodiment, the VHH antibody is recombinantly produced in a bacterium, e.g., *E. coli* or *Bacillus.* For example, expression in a bacterium may involve cytoplasmic and/or periplasmic expression and purification of the VHH antibody from the host cell, or secretory expression and purification of the VHH antibody from the culture medium. In certain embodiments, the nucleic acid sequence encoding the VHH antibody is fused to at least one sequence directing the expression to the periplasm and/or into the culture medium.

In a further particular embodiment, the VHH antibody is recombinantly produced in a eukaryotic host cell or host organism, preferably in yeast, e.g., in *Pichia pastoris*, *Saccharomyces cerevisiae*, *Schizosaccharomyces pombe*, or *Hansenula polymorpha*, or in an animal cell, particularly in an insect cell, e.g., an Sf-9 cell, or a mammalian cell, e.g., human or a hamster cell such as HEK293F, CHO or COS-7. For example, expression in a eukaryotic host cell or host organism, e.g., yeast may involve cytoplasmic and/or periplasmic expression and purification of the VHH antibody from the host cell, or preferably secretion from the host cell and purification of the VHH antibody from the culture medium. In certain embodiments, the nucleic acid sequence encoding the VHH antibody is fused to at least one sequence directing the expression into the culture medium.

### Medical applications

Still a further aspect of the present invention is the use of a VHH antibody as described above in medicine, particularly for therapeutic and/or in vitro or vivo diagnostic use. In certain embodiments, the VHH antibody is used in human medicine.

The VHH antibody of the present invention is useful in the prevention, treatment and/or mitigation of a condition caused by, associated with and/or accompanied by an activity of basigin. In certain embodiments, the activity of basigin is related to its receptor function, particularly on its receptor function on erythrocytes

Specific applications include the therapeutic and/or prophylactic treatment of malaria, particularly tropical malaria, by preventing the entry of *Plasmodium falciparum* merozoites into erythrocytes, through preventing the interaction of the plasmodial PfRH5 protein with basigin on the surface of erythrocytes. In particular embodiments, the VHH antibody of the present invention is used in a method for the therapeutic and/or prophylactic treatment of a P. falciparum infection. In further particular embodiments, the VHH antibody of the present invention is used in a method of reducing the severity of a P. falciparum infection.

Further applications include the therapeutic and/or prophylactic treatment of another condition caused by, associated with and/or accompanied by an infection with a pathogen that invades mammalian cells, e.g., erythrocytes, by interaction with basigin. In certain embodiments, the pathogen is a bacterium including but not limited to *Listeria monocytogenes* (Till et al., 2008); (Dhanda et al., 2019) and *Neisseria meningitidis* (Maïssa et al., 2017); (Bernard et al., 2014) or a virus that uses basigin as a receptor or co-receptor.

Still further applications include the modulation of immune and/or inflammatory responses by blocking receptor functions of basigin (Zhu et al., 2014), antagonizing cancer-promoting functions of basigin (Weidle et al., 2010), including but not limited to the induction of matrix metalloproteinases, tumor cell invasion, angiogenesis, and/or blocking the interaction of basigin with monocarboxylate transporters (MCTs), thereby preventing the export of lactate and thereby contributing to tumor cell death. Still further applications include antagonizing cardiovascular disorders including but not limited to atherosclerosis and myocardial infarction, e.g. by blocking the pro-inflammatory and thrombocyte-aggregating function of basigin (von Ungern-Sternberg et al., 2018).

In therapeutic applications, the VHH antibody is administered in an effective amount to a subject in need thereof, particularly to a human subject. The dose will depend on the specific type of agent, e.g., monovalent VHH antibody or multimeric VHH antibody, the type of disease, and the route of administration.

Typically, the VHH antibody is administered as a pharmaceutical composition comprising the active agent and a pharmaceutically acceptable carrier or excipient. Examples of suitable carriers and excipients for formulating antibodies or antibody fragments are well-known in the art.

Depending on the stage and the severity of the disorder, the pharmaceutical composition may be administered once or several times during the course of the disorder. For example, it may be administered once or several times daily, each second day, two times weekly or weekly for a suitable period of time.

In certain embodiments, the pharmaceutical composition is administered parenterally, e.g., by subcutaneous, intramuscular, or intravenous, intramuscular or subcutaneous injection or by infusion. In further embodiments, the pharmaceutical composition is administered locally, e.g., topically, orally, nasally, for example by spraying or intrapulmonary, for example by inhalation as an aerosol.

The VHH antibody may be administered alone as a monotherapy or together, e.g., sequentially, or simultaneously, with a further active agent as a combination therapy, particularly with a further agent that is useful in the prevention, treatment, and/or mitigation of a disorder caused by and/or associated with an activity of basigin, e.g., as listed above. In certain embodiments, the VHH antibody is administered in combination with an anti-malaria agent such as such as atovaquone, and/or pyrimethamine.

### Diagnostic and research applications

Diagnostic and research applications include in vitro methods wherein a VHH antibody or a set of different VHH antibodies is used for detecting, labelling and/or immobilizing erythrocytes in a sample, e.g., in a body fluid such as saliva, sputum, a lung lavage, a swab, blood, serum or plasma, stool samples or in a tissue or biopsy sample.

Diagnostic applications further include in vivo methods wherein a VHH antibody is used for detecting, labelling and/or immobilizing erythrocytes in a subject, particularly in a human patient. For diagnostic applications, the VHH antibody may carry a label for direct detection or used in combination with secondary detection reagents, e.g., biotin/streptavidin, detection antibodies, including conventional antibodies or VHH antibodies, for indirect detection according to established techniques in the art.

The present invention encompasses the use of VHH antibodies for targeting basigin with high sensitivity. The availability of three complementary epitope classes can be exploited for highly sensitive detection. In certain embodiments, the detection format comprises the binding of at least 2 different VHH antibodies, e.g., 2 or 3 different VHH antibodies to a basigin molecule, particularly the binding of at least 2 different VHH antibodies, e.g., 2 or 3 VHH antibodies each directed against a different epitope. In particular embodiments, the detection comprises the use of a set of 2 or 3 different VHH antibodies as described above.

In particular embodiments, the detection comprises a sandwich assay, e.g., a sandwich ELISA. In such a test format, a first VHH antibody may be immobilized to a solid phase for capturing basigin from the sample, and a second VHH antibody is employed in labelled form for the actual detection.

Further, the present invention is explained in more detail by the following Figures and Examples.

### Figure Legends

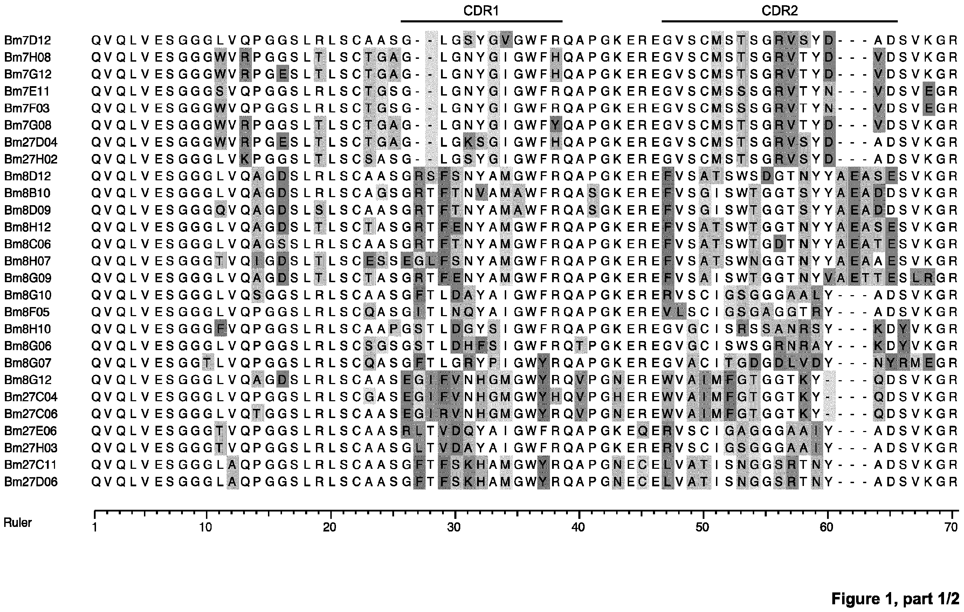

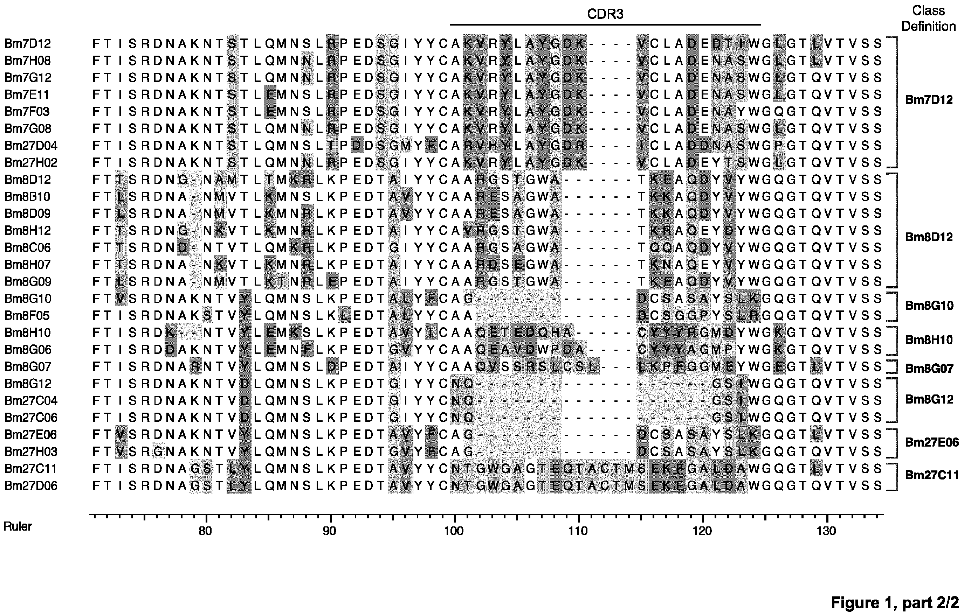

**Figure 1****: Sequence alignment and highlighting of variable regions**
   Figure 1 shows an alignment of sequences from selected VHH antibodies listed in Table 2. Residues that deviate from the consensus are highlighted by a gray background. The three variable CDR regions are indicated.
Figure 2: Affinities of VHH antibodies for basigin as measured by Bio-layer interferometry (BLI)
   Bio-layer interferometry (BLI) experiments were performed for basigin with the indicated VHH antibodies. Curves (grey) were fitted with a mass transport model (black). Calculated dissociation constants (K_{D}s) are shown above the curves.
**Figure 3****: Epitope binning of anti-basigin VHH antibodies revealed three distinct basigin-binding sites**
   (A) Epitope binning experiments were performed pairwise for the indicated VHH antibodies with a biotinylated basigin. When basigin was saturated with Bm7D12, Bm8D12, Bm8G10, Bm8H10, Bm8G06 and Bm27E06 were not able to bind further, suggesting its binding site was blocked by Bm7D12. Binding of other VHH class antibodies were not affected. Similar measurements were carried out for all VHH classes pairwise.
   (B) Representatives of three distinct epitope VHHs were allowed to bind immobilized basigin sequentially.

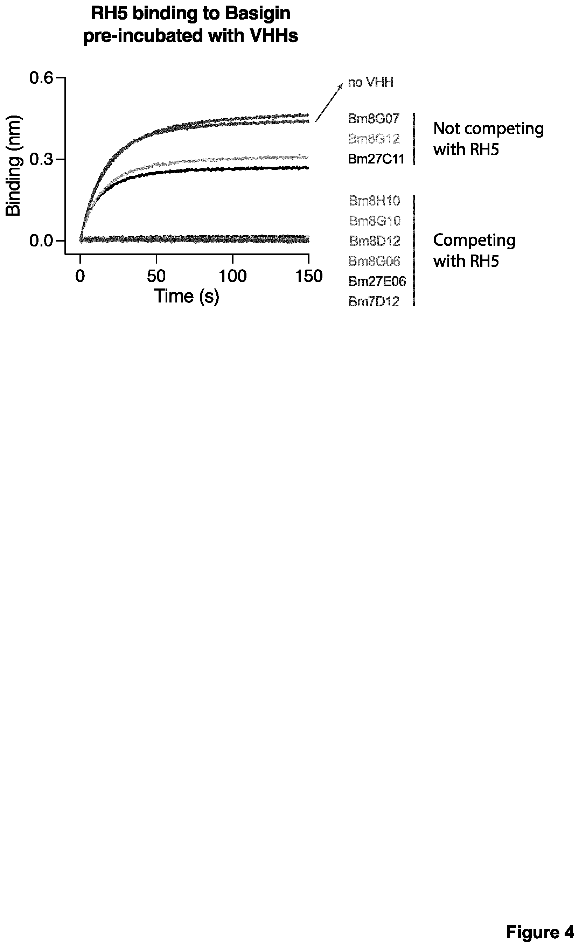

**Figure 4****: Competition of VHH antibodies with PfRH5 for Basigin binding**
   Competition experiments were performed for the indicated VHH antibodies with a biotinylated basigin. VHH antibodies were first allowed to bind to immobilized basigin, and further binding of PfRh5 was assessed.

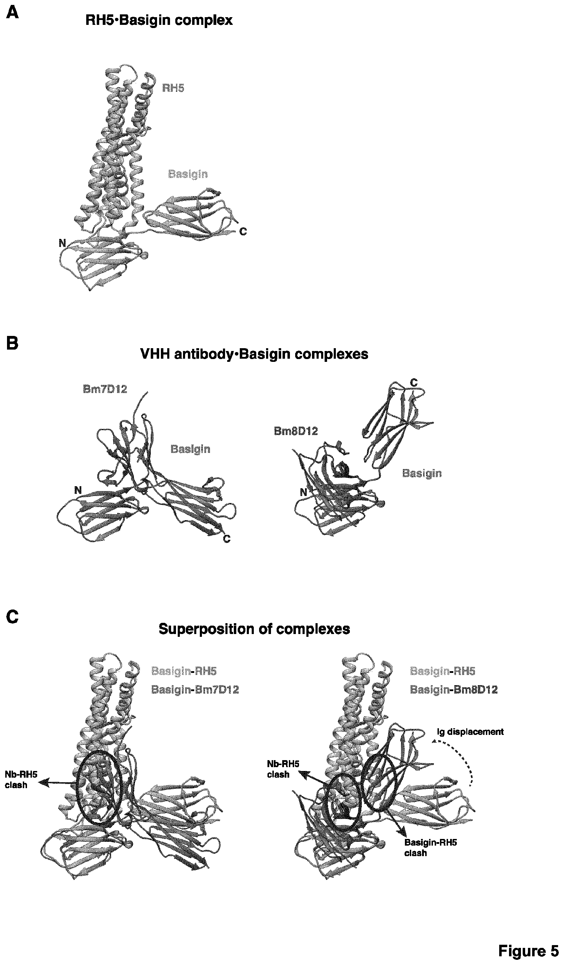

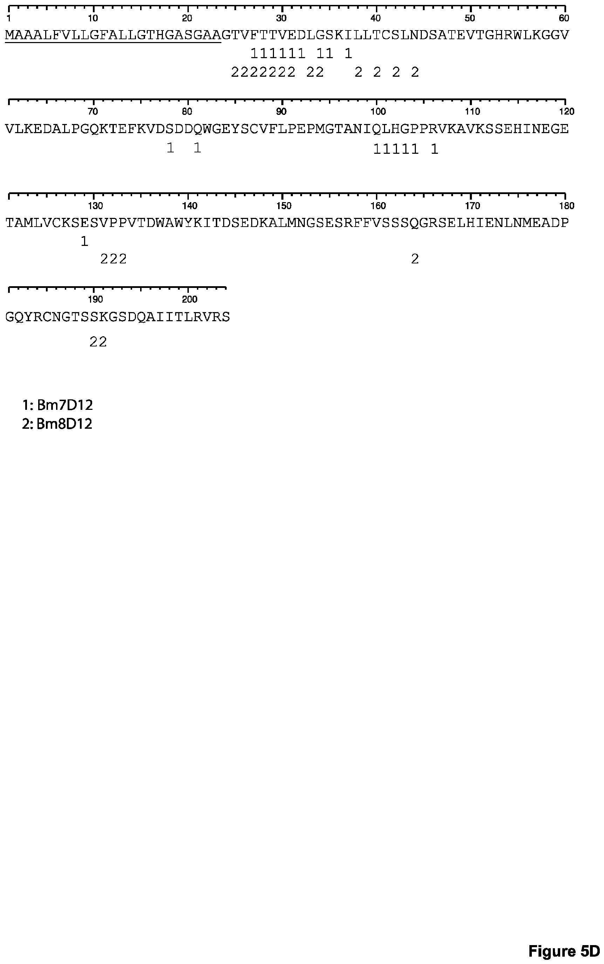

**Figure 5****: Crystal structure of the Epitope 1-binder Bm7D12 bound to a basigin dimer**
   (A) Crystal structure of the PfRH5 in complex with basigin in ribbon representation (PDB ID 4U0Q, (Wright et al., 2014)). Second PfRH5•basigin complex copy (chains C and D) was removed for clarity. N- and C termini of basigin were denoted with **N** and **C**, respectively.
   (B) Crystal structure of the tetrameric VHH antibody•basigin complexes in ribbon representation: (Left) Bm7D12 complex and (Right) Bm8D12 complex.
   (C) PfRH5 was docked onto the basigin dimers, based on the alignment of basigin N-terminal Ig domain with the PfRh5•basigin complex.
   (D) Sequence of basigin with the epitope of VHH Bm7D12 being denoted by "1" shown underneath and epitope of Bm8D12 denoted by "2". The underlined part of the sequence refers to the signal peptide that is cleaved off in the mature protein (isoform 2).

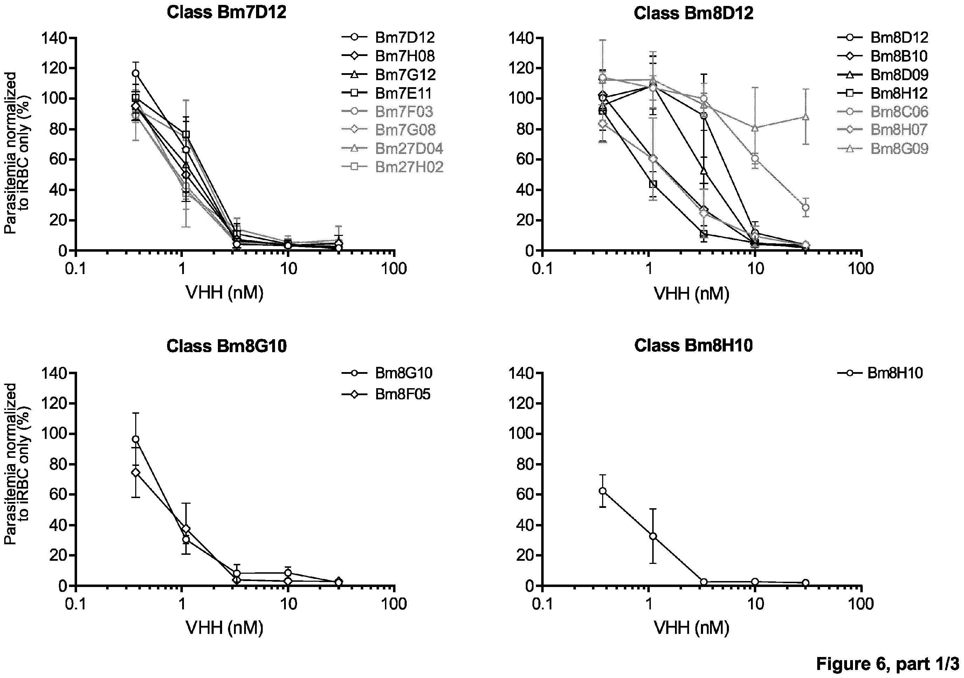

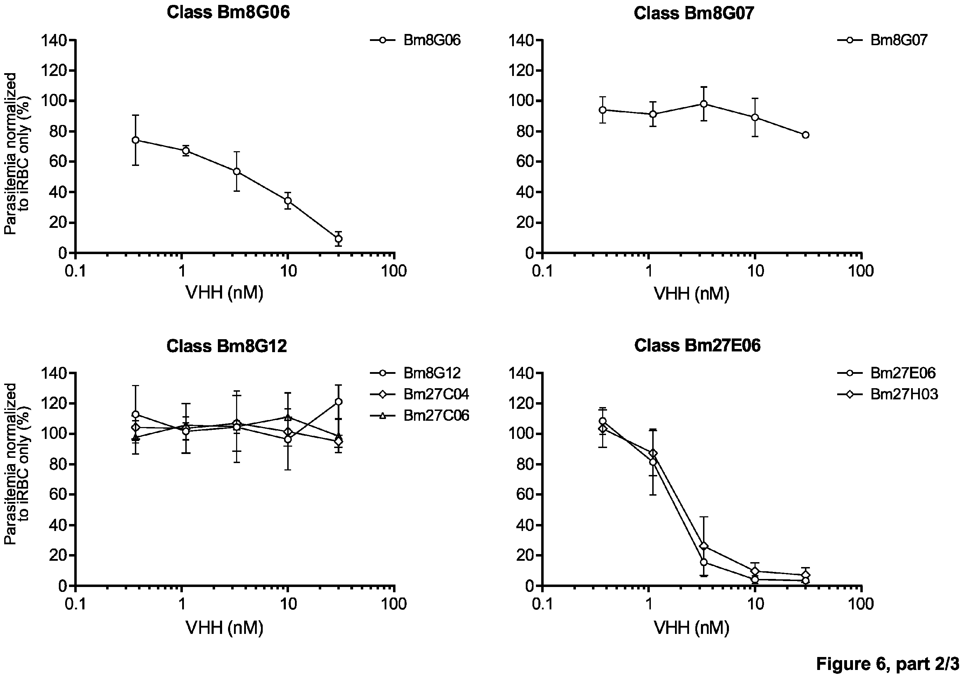

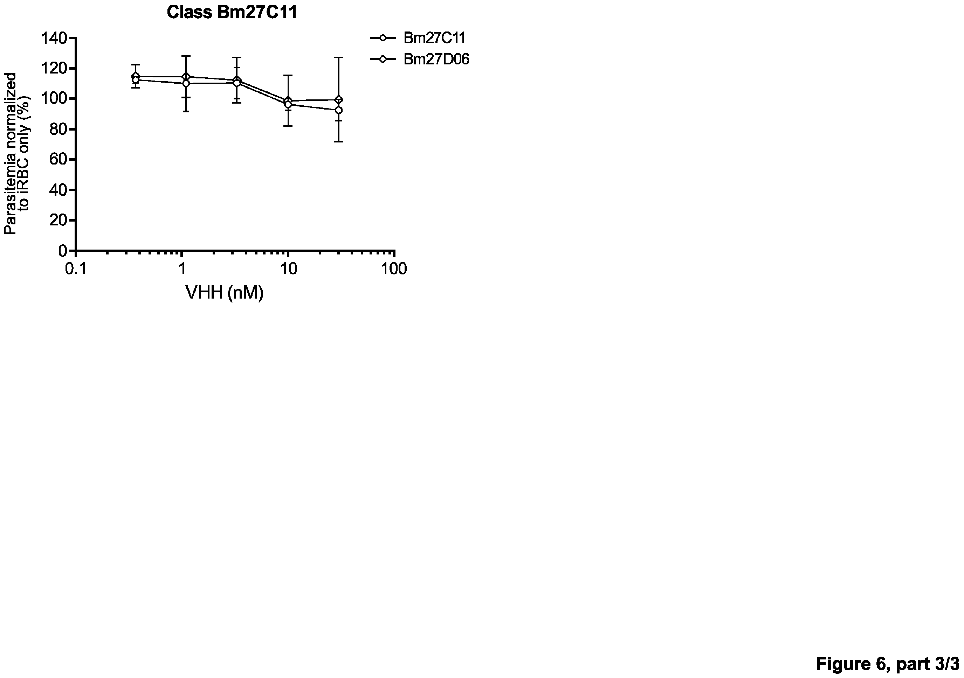

**Figure 6****: Blocking *Plasmodium falciparum* invasion and growth by anti-basigin VHH antibodies**
   Pf3D7 ring stage parasites were grown in the presence of the indicated anti-basigin VHH antibodies in three-fold dilutions for 72 h to complete one growth cycle. RBCs were fluorescently stained with Cell Mask Orange and parasite DNA with Hoechst with subsequent microscopy and automated counting of parasitized RBCs. The parasitemia of each VHH dilution was determined and expressed as percentage of the parasitemia of iRBCs only. Graphs are grouped according to the VHH classes. All VHH labelled with an asterisk show block of invasion, i.e. basigin neutralization down to 3 nM. Data represent the mean±SD from three independent experiments performed in triplicate.

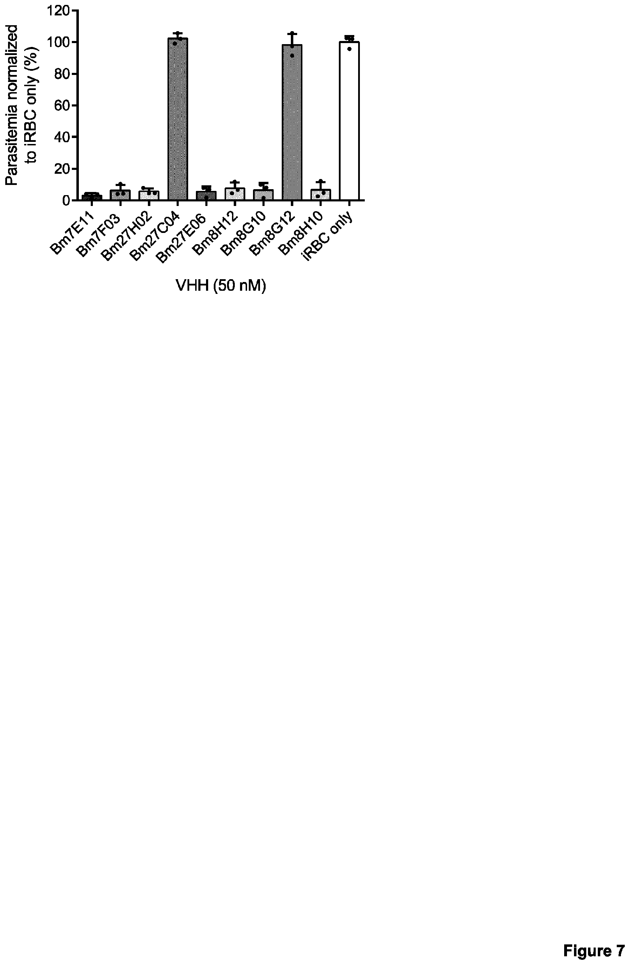

**Figure 7****: Prevention of parasite entry to RBCs by anti-basigin VHH antibodies**
   Pf3D7 mature stages (schizonts) were magnetically enriched and then further cultivated for 48 h in the presence of 50 nM of the indicated anti-basigin VHH antibody. During this time the schizonts burst and the next invasion cycle occurred. Parasitemia was determined by fluorescent staining of RBCs with Cell Mask Orange and of parasite DNA with Hoechst with subsequent microscopy and automated counting of parasitized RBCs. Data represent the mean±SD from one experiment performed in triplicate.

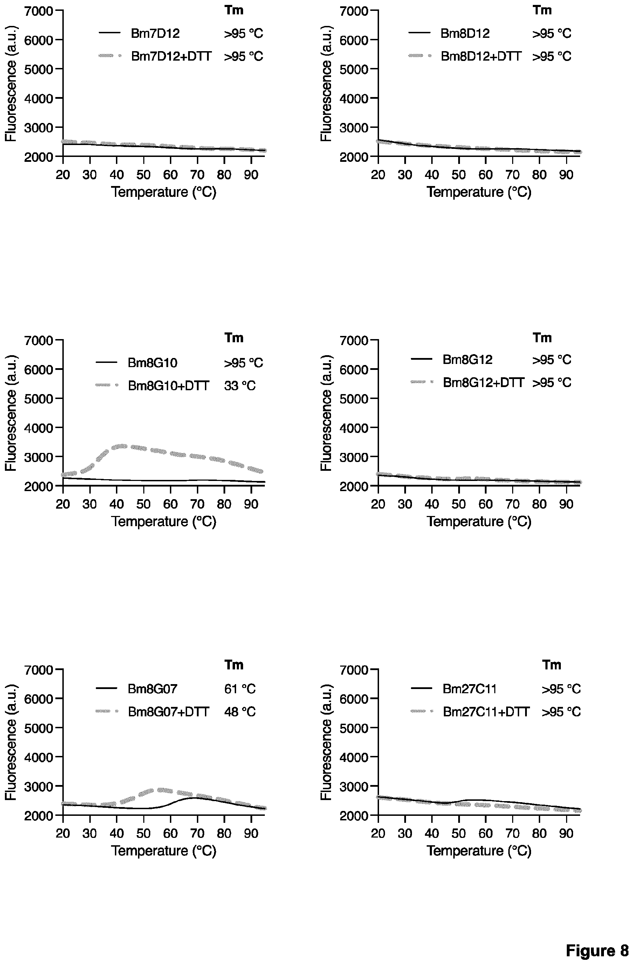

**Figure 8****: Thermal stability of VHH antibodies**
   VHH antibodies were subjected to Differential scanning fluorimetry (DSF) as detailed in Example 6. Thermal unfolding is here measured as an enhanced fluorescence of added SYPRO Orange following a stepwise increase in temperature with 532 nm excitation and a 555 nm long pass filter. Melting temperatures are defined as the inflection point of the first melting peak.

### Sequences of basigin and PfRH5 used herein

>Basigin Isoform 2 ectodomain (UniProt P35613-2, residues 24-204) (SEQ ID NO: 1) 
GTVFTTVEDLGSKILLTCSLNDSATEVTGHRWLKGGVVLKEDALPGQKTEFKVDSDDQWGEYSCVFL
PEPMGTANIQLHGPPRVKAVKSSEHINEGETAMLVCKSESVPPVTDWAWYKITDSEDKALMNGSESR
FFVSSSQGRSELHIENLNMEADPGQYRCNGTSSKGSDQAIITLRVRS
>Basigin used in BLI experiments (SEQ ID NO: 2) 
GSECSSGSEPGTVFTTVEDLGSKILLTCSLNDSATEVTGHRWLKGGVVLKEDALPGQKTEFKVDSDD
QWGEYSCVFLPEPMGTANIQLHGPPRVKAVKSSEHINEGETAMLVCKSESVPPVTDWAWYKITDSED
KALMNGSESRFFVSSSQGRSELHIENLNMEADPGQYRCNGTSSKGSDQAIITLRVRSC
>Basigin used in crystallography (SEQ ID NO: 3) 
GSEGSSGSEPGTVFTTVEDLGSKILLTCSLNDSATEVTGHRWLKGGVVLKEDALPGQKTEFKVDSDD
QWGEYSCVFLPEPMGTANIQLHGPPRVKAVKSSEHINEGETAMLVCKSESVPPVTDWAWYKITDSED
KALMNGSESRFFVSSSQGRSELHIENLNMEADPGQYRCNGTSSKGSDQAIITLRVRS
>PfRH5 (UniProt Q8IFM5, residues 25-526; Glycosylation mutant (Crosnier et al., 2013)) (SEQ ID NO: 4)

FENAIKKTKNQENNLALLPIKSTEEEKDDIKNGKDIKKEIDNDKENIKTNNAKDHSTYIKSYLNTNVNDG
LKYLFIPSHNSFIKKYSVFNQINDGMLLNEKNDVKNNEDYKNVDYKNVNFLQYHFKELSNYNIANSIDIL
QEKEGHLDFVIIPHYTFLDYYKHLSYNSIYHKSSTYGKCIAVDAFIKKINEAYDKVKSKCNDIKNDLIATIK

KLEHPYDINNKNDDSYRYDISEEIDDKSEETDDETEEVEDSIQDTDSNHAPSNKKKNDLMNRAFKKM
MDEYNTKKKKLIKCIKNHENDFNKICMDMKNYGTNLFEQLSCYNNNFCNTNGIRYHYDEYIHKLILSVK
SKNLNKDLSDMTNILQQSELLLTNLNKKMGSYIYIDTIKFIHKEMKHIFNRIEYHTKIINDKTKIIQDKIKLNI
WRTFQKDELLKRILDMSNEYSLFITSDHLRQMLYNTFYSKEKHLNNIFHHLIYVLQMKFNDVPIKMEYF
QTYKKNKPLTQ

### VHH antibody sequences

>Bm7D12 (SEQ ID NO: 5) 
QVQLVESGGGLVQPGGSLRLSCAASGLGSYGVGWFRQAPGKEREGVSCMSTSGRVSYDAD
SVKGRFTISRDNAKNTSTLQMNSLRPEDSGIYYCAKVRYLAYGDKVCLADEDTIWGLGTL
VTVSS
>Bm7H08 (SEQ ID NO: 9) 
QVQLVESGGGWVRPGGSLTLSCTGAGLGNYGIGWFHQAPGKEREGVSCMSTSGRVTYDVD
SVKGRFTISRDNAKNTSTLQMNNLRPEDSGIYYCAKVRYLAYGDKVCLADENASWGLGTL
VTVSS
>Bm7G12 (SEQ ID NO: 13) 
QVQLVESGGGWVRPGESLTLSCTGAGLGNYGIGWFHQAPGKEREGVSCMSTSGRVTYDVD
SVKGRFTISRDNAKNTSTLQMNNLRPEDSGIYYCAKVRYLAYGDKVCLADENASWGLGTQ
VTVSS
>Bm7E11 (SEQ ID NO: 17) 
QVQLVESGGGSVQPGGSLRLSCTGSGLGNYGIGWFRQAPGKEREGVSCMSSSGRVTYNVD
SVEGRFTISRDNAKNTSTLEMNSLRPEDSGIYYCAKVRYLAYGDKVCLADENASWGLGTQ
VTVSS
>Bm7F03 (SEQ ID NO: 21) 
QVQLVESGGGWVQPGGSLRLSCTGSGLGNYGIGWFRQAPGKEREGVSCMSSSGRVTYNVD
SVEGRFTISRDNAKNTSTLEMNSLRPEDSGIYYCAKVRYLAYGDKVCLADENAYWGQGTQ
VTVSS
>Bm7G08 (SEQ ID NO: 25) 
QVQLVESGGGWVRPGGSLTLSCTGAGLGNYGIGWFYQAPGKEREGVSCMSTSGRVTYDVD
SVKGRFTISRDNAKNTSTLQMNNLRPEDSGIYYCAKVRYLAYGDKVCLADENASWGLGTQ
VTVSS
>Bm27D04 (SEQ ID NO: 29) 
QVQLVESGGGWVRPGESLTLSCTGAGLGKSGIGWFHQAPGKEREGVSCMSTSGRVSYDAD
SVKGRFTISRDNAKNTSTLQMNSLTPDDSGMYFCARVHYLAYGDRICLADDNASWGPGTQ
VTVSS
>Bm27H02 (SEQ ID NO: 33) 
QVQLVESGGGLVKPGGSLTLSCSASGLGSYGIGWFRQAPGKEREGVSCMSTSGRVSYDAD
SVKGRFTISRDNAKNTSTLQMNNLRPEDSGIYYCAKVRYLAYGDKVCLADEYTSWGLGTQ
VTVSS
>Bm8D12 (SEQ ID NO: 37) 
QVQLVESGGGLVQAGDSLRLSCAASGRSFSNYAMGWFRQAPGKEREFVSATSWSDGTNYY
AEASESVKGRFTTSRDNGNAMTLTMKRLKPEDTAIYYCAARGSTGWATKEAQDYVYWGQG
TQVTVSS
>Bm8B10 (SEQ ID NO: 41) 
QVQLVESGGGLVQAGDSLRLSCAGSGRTFTNVAMAWFRQASGKEREFVSGISWTGGTSYY
AEADDSVKGRFTLSRDNANMVTLKMNSLKPEDTAVYYCAARESAGWATKKAQDYVYWGQG
TQVTVSS
>Bm8D09 (SEQ ID NO: 45) 
QVQLVESGGGQVQAGDSLSLSCAASGRTFTNYAMAWFRQASGKEREFVSGISWTGGTSYY
AEADDSVKGRFTLSRDNANMVTLKMNRLKPEDTAVYYCAARESAGWATKKAQDYVYWGQG
TQVTVSS
>Bm8H12 (SEQ ID NO: 49) 
QVQLVESGGGLVQAGDSLTLSCTASGRTFENYAMGWFRQAPGKEREFVSATSWTGGTNYY
AEASESVKGRFTTSRDNGNKVTLKMNRLKPEDTAIYYCAVRGSTGWATKRAQEYDYWGQG
TQVTVSS
>Bm8C06 (SEQ ID NO: 53) 
QVQLVESGGGLVQAGSSLRLSCAASGRTFTNYAMGWFRQAPGKEREFVSATSWTGDTNYY
AEATESVKGRFTTSRDNDNTVTLQMKRLKPEDTGIYYCAARGSAGWATQQAQDYVYWGQG
TQVTVSS
>Bm8H07 (SEQ ID NO: 57) 
QVQLVESGGGTVQIGDSLTLSCESSEGLFSNYAMGWFRQAPGKEREFVSATSWNGGTNYY
AEAAESVKGRFTTSRDNANKVTLKMNRLKPEDTAIYYCAARDSEGWATKNAQEYVYWGQG
TQVTVSS
>Bm8G09 (SEQ ID NO: 61) 
QVQLVESGGGLVQAGDSLTLSCTASGRTFENYAMGWFRQAPGKEREFVSAISWTGGTNYV
AETTESLRGRFTLSRDNANMVTLKTNRLEPEDTAIYYCAARGSTGWATKEAQDYVYWGQG
TQVTVSS
>Bm8G10 (SEQ ID NO: 65) 
QVQLVESGGGLVQSGGSLRLSCAASGFTLDAYAIGWFRQAPGKERERVSCIGSGGGAALY
ADSVKGRFTVSRDNAKNTVYLQMNSLKPEDTALYFCAGDCSASAYSLKGQGTQVTVSS
>Bm8F05 (SEQ ID NO: 69) 
QVQLVESGGGLVQPGGSLRLSCQASGITLNQYAIGWFRQAPGKEREVLSCIGSGAGGTRY
ADSVKGRFTISRDNAKSTVYLQMNSLKLEDTALYYCAADCSGGPYSLRGQGTQVTVSS
>Bm8H10 (SEQ ID NO: 73) 
QVQLVESGGGFVQPGGSLRLSCAAPGSTLDGYSIGWFRQAPGKEREGVGCISRSSANRSY
KDYVKGRFTISRDKNTVYLEMKSLKPEDTAVYICAAQETEDQHACYYYRGMDYWGKGTQV
TVSS
>Bm8G06 (SEQ ID NO: 77) 
QVQLVESGGGLVQPGGSLRLSCSGSGSTLDHFSIGWFRQTPGKEREGVGCISWSGRNRAY
KDYVKGRFTISRDDAKNTVYLEMNFLKPEDTGVYYCAAQEAVDWPDACYYYAGMPYWGKG
TQVTVSS
>Bm8G07 (SEQ ID NO: 81) 
QVQLVESGGTLVQPGGSLRLSCQASGFTLGRYPIGWYRQAPGKEREGVACITGDGDLVDY
NYRMEGRFTISRDNARNTVYLQMNSLDPEDTAIYYCAAQVSSRSLCSLLKPFGGMEYWGE
GTLVTVSS
>Bm8G12 (SEQ ID NO: 85) 
QVQLVESGGGLVQAGDSLRLSCAASEGIFVNHGMGWYRQVPGNEREWVAIMFGTGGTKYQ
DSVKGRFTISRDNAKNTVDLQMNSLKPEDTGIYYCNQGSIWGQGTQVTVSS
>Bm27C04 (SEQ ID NO: 89) 
QVQLVESGGGLVQPGGSLRLSCGASEGIFVNHGMGWYHQVPGHEREWVAIMFGTGGTKYQ
DSVKGRFTISRDNAKNTVDLQMNSLKPEDTGIYYCNQGSIWGQGTQVTVSS
>Bm27C06 (SEQ ID NO: 93) 
QVQLVESGGGLVQTGGSLRLSCAASEGIRVNHGMGWYRQVPGNEREWVAIMFGTGGTKYQ
DSVKGRFTISRDNAKNTVDLQMNSLKPEDTGIYYCNQGSIWGQGTQVTVSS
>Bm27E06 (SEQ ID NO: 97) 
QVQLVESGGGTVQPGGSLRLSCAASRLTVDQYAIGWFRQAPGKEQERVSCIGAGGGAAIY
ADSVKGRFTVSRDNAKNTVYLQMNSLKPEDTAVYFCAGDCSASAYSLKGQGTLVTVSS
>Bm27H03 (SEQ ID NO: 101 
QVQLVESGGGTVQPGGSLRLSCAASGLTVDAYAIGWFRQAPGKERERVSCIGSGGGAAIY
ADSVKGRFTVSRGNAKNTVYLQMNSLKPEDTGVYFCAGDCSASAYSLKGQGTQVTVSS
>Bm27C11 (SEQ ID NO: 105 
QVQLVESGGGLAQPGGSLRLSCAASGFTFSKHAMGWYRQAPGNECELVATISNGGSRTNY
ADSVKGRFTISRDNAGSTLYLQMNSLKPEDTAVYYCNTGWGAGTEQTACTMSEKFGALDA
WGQGTLVTVSS
>Bm27D06 (SEQ ID NO: 109)

QVQLVESGGGLAQPGGSLRLSCAASGFTFSKHAMGWYRQAPGNECELVATISNGGSRTNY
ADSVKGRFTISRDNAGSTLYLQMNSLKPEDTAVYYCNTGWGAGTEQTACTMSEKFGALDA
WGQGTQVTVSS

### Examples

A convenient source of single-domain antibodies (VHHs, nanobodies) consists in camelid animals, in particular alpacas (Arbabi Ghahroudi et al., 1997). The antibody repertoire of these animals includes heavy-chain only antibodies, where the antigen binding site consists of a single peptide domain (Hamers-Casterman et al., 1993). In contrast to monoclonal IgGs, which are manufactured in mammalian cells, nanobodies can also be produced in bacteria or yeast. Apart from their low molecular weight, certain nanobodies proved to be hyper-thermostable and display a particularly high affinity (Güttler et al., 2021).

The primary aim was to obtain VHH antibodies that bind basigin at the erythrocyte surfaces and block interaction with *Plasmodium falciparum PfRH5* (PfRH5). To this end, the inventors recombinantly produced basigin isoform 2 (expressed in erythrocytes) ectodomain (i.e., the domain that extends into the extracellular space) and immunized two alpacas three times at three-weeks intervals.

One week after the final immunization, blood samples were taken, lymphocytes were isolated, and mRNAs were purified and reverse transcribed. VHH-coding regions were amplified by nested PCR and cloned as cDNAs into an M13 phagemid, yielding libraries with >100 million independent clones.

In the next steps, two rounds of phage display were performed using basigin as a bait. 192 selected clones were sequenced and classified by sequence similarity. 19 representatives of all VHH classes were then expressed in *E*. *coli* and purified (Bm7 and Bm8 series, see Figure 1 for specific sequences).

For generation of a "second generation" VHH antibodies, 16 months after the last immunization, the alpacas were immunized with basigin, followed by immune library preparation and phage display selection. This yielded additional VHH antibodies.

These include VHH variants of previously isolated classes (e.g., belonging to the Bm7D12 and Bm8G12 classes), and also, new VHH classes such as Bm27C11 and Bm27E04.

The inventors then analyzed VHH-Basigin interactions by Bio-Layer Interferometry (BLI; (Abdiche et al., 2008)). Results are shown in Figure 2 and in Table 1. These measurements revealed extremely high affinities (K_{D}s up to 10 pM, please note that the BLI setup does not allow to discern affinities below 10 pM) with high on-rates (mostly around 10⁵ - 10⁶ M⁻¹·s⁻¹), and non-detectable or nearly non-detectable dissociation rates for most of the VHHs.

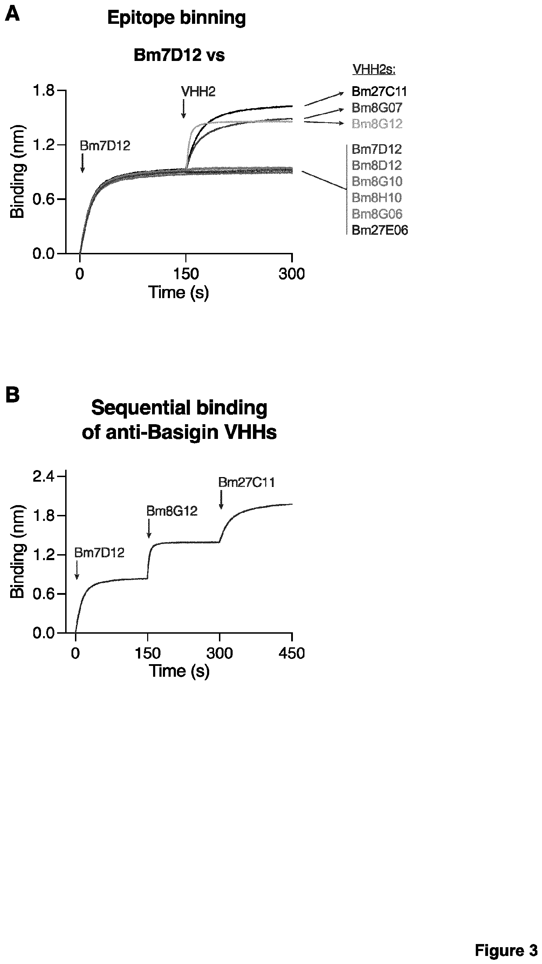

To assess if and how many complementary binding sites these VHHs have on the basigin molecule, the inventors performed binning experiments using BLI. In these experiments, one VHH antibody was allowed to bind to immobilized basigin, and then the binding of another VHH was monitored. An example is shown in Figure 3. When basigin was completely saturated with Bm7D12, then Bm7D12 itself as well as Bm8D12, Bm8G10, Bm8H10, Bm8G06 and Bm27E06 were not able to bind further, while the binding of Bm8G07, Bm8G12 and Bm27C11 remained unaffected. By repeating this setup with all representative VHH class members, 'Binning' experiments revealed three complementary epitopes (epitopes 1-3) for basigin (Table 1), where epitope 1-binding VHH antibodies could bind simultaneously with either epitope 2-binders or epitope 3-binders. Likewise, epitope 2- and 3-binders could also bind simultaneously. Figure 3 shows that up to three VHH antibodies can bind basigin simultaneously, provided they are chosen such that each recognizes a different, complementary epitope.

In the following experiments, the inventors tested by BLI whether these VHH antibodies could block Basigin-PfRH5 interaction. In these competition experiments, VHH antibodies were allowed to bind to immobilized basigin, and then the binding of PfRH5 was monitored. An example is shown in Figure 4. When VHH representatives of epitope 2- and 3-binders (i.e., Bm8G07, Bm8G12 and Bm27C11) bound basigin, the binding of PfRH5 was not affected, whereas no further binding was observed when epitope 1-binders (Bm7D12, Bm8D12, Bm8G06, Bm8G10, Bm8H10, Bm27E06) were allowed to bind basigin first. Overall, the inventors found that only epitope 1-binders were competing with PfRH5 for basigin binding (Table 1).

To understand the structural basis of the PfRH5 competition, the inventors crystallized basigin•epitope 1-binder VHH complexes. The inventors solved the X-ray crystal structures of the basigin in complex with Bm7D12 or with Bm8D12 (Figure 5). The crystallographic analysis revealed that the PfRH5 block is caused in two ways, (i) by shielding of the binding site and (ii) by 'freezing' the linker between two basigin Ig domains in a conformation that is incompatible with PfRH5 binding.

In further experiments, the inventors monitored the capability of VHH antibodies to interfere with the parasite proliferation in RBCs. *Plasmodium falciparum* (strain 3D7) was grown in RBCs from human donors, and the parasites were synchronized through sorbitol treatment. Addition of epitope 1 anti-basigin VHHs prevented the propagation of plasmodia after finishing the first cycle of propagation, i.e. after the first schizont stage (Figure 6). This strongly suggests that anti-basigin VHH antibodies are indeed effective to prevent the spread of plasmodia in an RBC population.

The minimum concentration of nanobodies required for this activity was around 1 nM. The following calculation illustrates that this is roughly equivalent to the amount of basigin molecules in the RBC solution. A 2% haematocrit in the culture (as opposed to 50% in blood, with 5×10⁹ erythrocytes per L) corresponds to an RBC concentration of 2×10¹¹ /L. Since one RBC carries about 3000 basigin molecules (Reid et al., 2012), this corresponds to a concentration of 6×10¹⁴ molecules of basigin per litre, or 1 nM. Thus, the effective nanobody concentration is not limited by the affinity to basigin (which is actually in the picomolar range, Figure 2) but by the need to cover the majority of basigin molecules present in a suspension of RBCs.

The inventors then tested how exactly the nanobodies interfere with the growth and spread of plasmodia. They conducted invasion assays with magnetically purified schizonts. Adding epitope 1 anti-basigin VHHs to this preparation completely prevented the occurrence of invaded RBCs (Figure 7), strongly suggesting that it is indeed the entry step that is counteracted by the VHH antibodies.

For the intended therapeutic application, the anti-basigin VHH antibodies should not only bind their targets with high affinity, but they should also be developable as biological drugs. This includes that they are stable enough to survive a lengthy, large-scale production process as well as transportation and storage (ideally for years in formulation) without aggregation or loss of activity.

A good predictor of stability is thermostability, which can be measured, e.g., by thermal shift assays or specifically by differential scanning fluorimetry (Goldberg et al., 2011). The method exploits that melting (thermal unfolding) of a protein exposes aromatic/hydrophobic residues (from its hydrophobic core), which then bind and enhance the fluorescence of the added SYPRO Orange dye.

Figure 8 shows such analyses for the disclosed VHH antibodies, with slow heating from 20°C to 95°C. The majority of VHHs (see Table 1) showed only a negligible unfolding signal and thus no melting (the small fluorescence peak did not exceed or hardly exceeded the measured fluorescence at 20°C). The very low melting amplitudes indicate resistance to melting and thus full thermal stability. We observed that most of the VHHs retained their thermostability even in the presence of the disulfide bond-reducing agent DTT (dithiothreitol) (Figure 9 and Table 1).

### Example 1: Kinetic measurements by Bio-layer interferometry (BLI)

For affinity measurements, basigin was produced with N- and C- terminal ectopic cysteines and biotinylated via attachment of sulfhydryl-reactive maleimide-PEG11-biotin (Thermo Scientific, 21911). It was immobilized at a concentration of 100 nM for 120 seconds on High Precision Streptavidin biosensors of an Octet RED96e instrument (ForteBio/Sartorius), using Phosphate-Buffered Saline (PBS) pH 7.4, 0.02% (w/v) Tween 20 and 0.1% (w/v) bovine serum albumin (BSA) as the assay buffer. 40, 20, 10, 5, 2.5 or 1.25 nM of the indicated VHH antibodies were then allowed to bind for 600 seconds, followed by a dissociation step of 1800 seconds. Binding and dissociation were recorded as wavelength shifts (in nm). Baselines were recorded by measuring a 'minus VHH control' in parallel. On-rates, off-rates, and dissociation constants (K_{D}s) were calculated using the Octet Data Analysis HT 12.0 software by fitting a mass transport model to the data. This revealed very tight binding of the analyzed VHHs to Basigin.

The results are summarized in Table 1 and shown in Figure 2.

### Example 2: Identification of complementary basigin epitopes

Epitope binning experiments were performed in a similar set up as described in Example 1. Biotinylated basigin was immobilized on biosensors. The biosensors were dipped into wells containing 250 nM VHH (VHH1) for 150 seconds, and then incubated for 150 seconds with another VHH (VHH2) at 250 nM concentration. When binding of the VHH2 is blocked in the presence of VHH1, they were considered same epitope binders. By repeating this for all VHH class representatives, VHH antibodies were grouped into three different epitope binders.

The results are shown in Table 1 and Figure 3. Bm7D12, Bm8G12 and Bm27C11, were able to bind immobilized Basigin sequentially.

### Example 3: Identification of PfRH5-blocking VHH antibodies

Competition experiments were performed in a similar set-up as described in Example 2. Biotinylated basigin was immobilized on biosensors. The biosensors were dipped into wells containing 250 nM VHH for 150 seconds, and then incubated for 150 seconds with PfRH5 at 300 nM concentration. If binding of PfRH5 is blocked in the presence of VHH antibody, they are considered competing antibodies. Unblocked PfRH5 binding was recorded by using assay buffer instead of a VHH.

The results are shown in Table 1 and Figure 4. Only epitope 1-binder VHH antibodies were competing with PfRH5 for basigin binding.

### Example 4: Structural characterization of basigin•Bm7D12 complex

To understand the structural basis of the PfRH5 competition, basigin was crystallized in complex with Bm7D12 and with Bm8D12, both epitope 1-binder VHH antibodies.

The X-ray crystal structures of tetrameric Basigin•Bm7D12 and Basigin•Bm8D12 were solved (Figure 5). Comparison of the structures with the PfRH5-bound basigin structure (Wright et al., 2014) revealed that both VHH antibodies block PfRH5-binding by directly competing for the basigin-binding site. Note that the PfRH5 clashes with the VHHs, explaining how the here disclosed epitope 1-binder VHH antibodies could block the binding of the PfRH5 to its receptor basigin.

### Example 5: Parasite culture

*P. falciparum* parasites (Pf3D7) were obtained from Hosam Shams-Eldin, Marburg University, Germany. Asexual blood stage parasites were cultivated *in vitro* in AB+ or AB-erythrocytes (German red cross blood bank, DRK-NSTOB) at 4% hematocrit in RPMI 1640 supplemented with 5.95 g/L HEPES, 50 mg/L Hypoxanthine, 0.2% NaHCOs, 0.5% AlbuMAX-I (Gibco) and 12.5 µg/mL Gentamicin. Cultures were maintained at 37°C in 95% N₂, 5% O₂, 5% CO₂. Parasites were synchronized by lysis of late stages in 5% sorbitol. To enrich parasites, synchronized mature (late trophozoite and schizont) stages ware passed over LD magnetic MACS columns (Miltenyi Biotech) to remove uninfected cells. Eluted RBCs (100 % parasitized) were diluted to 4% hematocrit with medium and the parasitemia adjusted to 0.5% for invasion assays.

### Example 6: Parasite invasion assay with VHH antibodies

Ring stage parasites at 0.5% parasitemia were grown in 100 µl culture medium at 2% hematocrit in 96 well microtiter plates (Greiner) in the presence of three-fold dilutions of each VHH antibody. Infected RBCs (iRBCs) without VHH served as control. After incubation for 72 h (one growth cycle), the cells were stained with Cell Mask Orange (Invitrogen) diluted 1:1000 in medium for 10 min at 37°C and then fixed with 4% Paraformaldehyde/0.075% Glutaraldehyde in PBS for 30 min at 37°C. The supernatants were discarded, and the parasite DNA stained with Hoechst 33342 (Invitrogen) in PBS. The cells were imaged in PBS using an inverted microscope (Zeiss CD7) equipped with a 20X (NA0.7) objective with 2X optovar (40X). Ten images per well were acquired and analyzed in an automated way using the Zeiss Zen Image Analysis tool. Object identification was based on Cell Mask Orange (RBCs) and Hoechst (parasites) and at least 5000 RBCs per condition were analyzed. The parasitemia of each well was determined (Number of iRBCs / total number of cells). For comparison, the parasitemia was expressed as percentage of the parasitemia of iRBCs only. All samples were measured in triplicate in at least three independent replicates.

### Example 7: Thermostability measurement of VHH antibodies

VHH antibodies were subjected to Differential scanning fluorimetry (DSF), which exploits that thermal unfolding exposes aromatic/hydrophobic residues, which then bind and enhance the fluorescence of the added SYPRO Orange dye. Assays were performed in a volume of 20 µl, at 1 mg/ml VHH concentration in 50 mM Tris/HCl, 150 mM NaCl (pH 8.0 at 20°C), and 1x dye (diluted from a 5000x stock; Life Technologies), in the absence or presence of 10 mM DTT (dithiothreitol). Two sample replicates were pipetted into a Hard-Shell^{®} 96-well plate (Bio-Rad). The plate was sealed with transparent MicroSeal^{®} 'B' Seal (Bio-Rad), briefly centrifuged to remove air bubbles, and placed in a CFX96 Real-Time System (C1000 Thermal Cycler, BioRad). The samples were incubated for 5 min at 20°C and then heated in 1°C increments of 45 seconds up to 95°C. Fluorescence was measured at the end of each step with 532 nm excitation and a 555 nm long pass filter. Melting temperatures are defined as the inflection point of the first melting peak.

The results are shown in Table 1 and Figure 8. VHH antibodies Bm8C06, Bm8G06, and Bm8G07 melt already at 61-63°C, while majority of VHH antibodies showed only a negligible melting amplitude and thus remained stable throughout 95°C. Remarkably, Bm7D12, Bm7H08, Bm8D12, Bm8H07, Bm8G12, and Bm27C11 were resistant to melting even in the presence of disulfide-reducing DTT.

### References

Abdiche, Y., Malashock, D., Pinkerton, A., & Pons, J. (2008). Determining kinetics and affinities of protein interactions using a parallel real-time label-free biosensor, the Octet. Anal Biochem, 377(2), 209-217. https://doi.org/10.1 016/i.ab.2008.03.035
Alanine, D. G. W., Quinkert, D., Kumarasingha, R., Mehmood, S., Donnellan, F. R., Minkah, N. K., Dadonaite, B., Diouf, A., Galaway, F., Silk, S. E., Jamwal, A., Marshall, J. M., Miura, K., Foquet, L., Elias, S. C., Labbé, G. M., Douglas, A. D., Jin, J., Payne, R. O., ... Draper, S. J. (2019). Human Antibodies that Slow Erythrocyte Invasion Potentiate Malaria-Neutralizing Antibodies. Cell, 178(1), 216-228.e21. https://doi.org/10.1016/j.cell.2O19.05.025
Arbabi Ghahroudi, M., Desmyter, A., Wyns, L., Hamers, R., & Muyldermans, S. (1997). Selection and identification of single domain antibody fragments from camel heavy-chain antibodies. FEBS Lett, 414(3), 521-526. https://doi.ora/10.1016/s0014-5793(97)01062-4
Beeson, J. G., Kurtovic, L., Valim, C., Asante, K. P., Boyle, M. J., Mathanga, D., Dobano, C., & Moncunill, G. (2022). The RTS,S malaria vaccine: Current impact and foundation for the future. Sci Transl Med, 14(671), eabo6646. https://doi.org/10.1126/scitranslmed.abo6646
Bernard, S. C., Simpson, N., Join-Lambert, O., Federici, C., Laran-Chich, M. P., Maïssa, N., Bouzinba-Ségard, H., Morand, P. C., Chretien, F., Taouji, S., Chevet, E., Janel, S., Lafont, F., Coureuil, M., Segura, A., Niedergang, F., Marullo, S., Couraud, P. O., Nassif, X., ... Bourdoulous, S. (2014). Pathogenic Neisseria meningitidis utilizes CD147 for vascular colonization. Nat Med, 20(7), 725-731. https://doi.org/10.1038/nm.3563
Björkman, A., Benn, C. S., Aaby, P., & Schapira, A. (2023). RTS,S/AS01 malaria vaccine-proven safe and effective. Lancet Infect Dis, 23(8), e318-e322. https://doi.org/10.1016/S1473-3099(23)00126-3
Boyle, M. J., Wilson, D. W., Richards, J. S., Riglar, D. T., Tetteh, K. K., Conway, D. J., Ralph, S. A., Baum, J., & Beeson, J. G. (2010). Isolation of viable Plasmodium falciparum merozoites to define erythrocyte invasion events and advance vaccine and drug development. Proc Natl Acad Sci U S A, 107(32), 14378-14383. https://doi.org/10.1073/pnas.1009198107
Chen, L., Xu, Y., Wong, W., Thompson, J. K., Healer, J., Goddard-Borger, E. D., Lawrence, M. C., & Cowman, A. F. (2017). Structural basis for inhibition of erythrocyte invasion by antibodies to Plasmodium falciparum protein CyRPA. Elife, 6, e21347. https://doi.org/10.7554/eLife.21347
Cowman, A. F., Tonkin, C. J., Tham, W. H., & Duraisingh, M. T. (2017). The Molecular Basis of Erythrocyte Invasion by Malaria Parasites. Cell Host Microbe, 22(2), 232-245. https://doi.org/10.1016/j.chom.2017.07.003
Crosnier, C., Wanaguru, M., McDade, B., Osier, F. H., Marsh, K., Rayner, J. C., & Wright, G. J. (2013). A library of functional recombinant cell-surface and secreted P. falciparum merozoite proteins. Mol Cell Proteomics, 12(12), 3976-3986. https://doi.org/10.1074/mcp.0113.028357
Dhanda, A. S., Lulic, K. T., Yu, C., Chiu, R. H., Bukrinsky, M., & Guttman, J. A. (2019). Listeria monocytogenes hijacks CD147 to ensure proper membrane protrusion formation and efficient bacterial dissemination. Cell Mol Life Sci, 76(20), 4165-4178. https://doi.org/10.1007/s00018-019-03130-4
Douglas, A. D., Baldeviano, G. C., Lucas, C. M., Lugo-Roman, L. A., Crosnier, C., Bartholdson, S. J., Diouf, A., Miura, K., Lambert, L. E., Ventocilla, J. A., Leiva, K. P., Milne, K. H., Illingworth, J. J., Spencer, A. J., Hjerrild, K. A., Alanine, D. G., Turner, A. V., Moorhead, J. T., Edgel, K. A., ... Draper, S. J. (2015). A PfRH5-based vaccine is efficacious against heterologous strain blood-stage Plasmodium falciparum infection in aotus monkeys. Cell Host Microbe, 17(1), 130-139. https://doi.org/10.1016/j.chom.2014.11.017
Douglas, A. D., Williams, A. R., Illingworth, J. J., Kamuyu, G., Biswas, S., Goodman, A. L., Wyllie, D. H., Crosnier, C., Miura, K., Wright, G. J., Long, C. A., Osier, F. H., Marsh, K., Turner, A. V., Hill, A. V., & Draper, S. J. (2011). The blood-stage malaria antigen PfRH5 is susceptible to vaccine-inducible cross-strain neutralizing antibody. Nat Commun, 2, 601. https://doi.org/10.1038/ncomms1615
Douglas, A. D., Williams, A. R., Knuepfer, E., Illingworth, J. J., Furze, J. M., Crosnier, C., Choudhary, P., Bustamante, L. Y., Zakutansky, S. E., Awuah, D. K., Alanine, D. G., Theron, M., Worth, A., Shimkets, R., Rayner, J. C., Holder, A. A., Wright, G. J., & Draper, S. J. (2014). Neutralization of Plasmodium falciparum merozoites by antibodies against PfRH5. J Immunol, 192(1), 245-258. https://doi.org/10.4049/jimmunol.1302045
Duffey, M., Blasco, B., Burrows, J. N., Wells, T. N. C., Fidock, D. A., & Leroy, D. (2021). Assessing risks of Plasmodium falciparum resistance to select next-generation antimalarials. Trends Parasitol, 37(8), 709-721. https://doi.org/10.1016/j.pt.2021.04.006
Favuzza, P., de Lera Ruiz, M., Thompson, J. K., Triglia, T., Ngo, A., Steel, R. W. J., Vavrek, M., Christensen, J., Healer, J., Boyce, C., Guo, Z., Hu, M., Khan, T., Murgolo, N., Zhao, L., Penington, J. S., Reaksudsan, K., Jarman, K., Dietrich, M. H., ... Cowman, A. F. (2020). Dual Plasmepsin-Targeting Antimalarial Agents Disrupt Multiple Stages of the Malaria Parasite Life Cycle. Cell Host Microbe, 27(4), 642-658.e12. https://doi.org/10.1016/j.chom.2020.02.005
Galaway, F., Yu, R., Constantinou, A., Prugnolle, F., & Wright, G. J. (2019). Resurrection of the ancestral PfRH5 invasion ligand provides a molecular explanation for the origin of P. falciparum malaria in humans. PLoS Biol, 17(10), e3000490. https://doi.org/10.1371/journal.pbio.3000490
Gilson, P. R., & Crabb, B. S. (2009). Morphology and kinetics of the three distinct phases of red blood cell invasion by Plasmodium falciparum merozoites. Int J Parasitol, 39(1), 91-96. https://doi.org/10.1016/j.iipara.2008.09.007
Goldberg, D. S., Bishop, S. M., Shah, A. U., & Sathish, H. A. (2011). Formulation development of therapeutic monoclonal antibodies using high-throughput fluorescence and static light scattering techniques: role of conformational and colloidal stability. J Pharm Sci, 100(4), 1306-1315. https://doi.org/10.1002/jps.22371
Güttler, T., Aksu, M., Dickmanns, A., Stegmann, K. M., Gregor, K., Rees, R., Taxer, W., Rymarenko, O., Schünemann, J., Dienemann, C., Gunkel, P., Mussil, B., Krull, J., Teichmann, U., Groβ, U., Cordes, V. C., Dobbelstein, M., & Görlich, D. (2021). Neutralization of SARS-CoV-2 by highly potent, hyperthermostable, and mutation-tolerant nanobodies. EMBO J, 40(19), e107985. https://doi.org/10.15252/embj.2021107985
Hadjilaou, A., Brandi, J., Riehn, M., Friese, M. A., & Jacobs, T. (2023). Pathogenetic mechanisms and treatment targets in cerebral malaria. Nat Rev Neurol, 19(11), 688-709. https://doi.org/10.1038/s41582-023-00881-4
Hamers-Casterman, C., Atarhouch, T., Muyldermans, S., Robinson, G., Hamers, C., Songa, E. B., Bendahman, N., & Hamers, R. (1993). Naturally occurring antibodies devoid of light chains. Nature, 363(6428), 446-448. https://doi.org/10.1038/363446a0
Hughes, K. R., Biagini, G. A., & Craig, A. G. (2010). Continued cytoadherence of Plasmodium falciparum infected red blood cells after antimalarial treatment. Mol Biochem Parasitol, 169(2), 71-78. https://doi.org/10.1016/j.molbiopara.2009.09.007
Kanjee, U., Gruring, C., Chaand, M., Lin, K. M., Egan, E., Manzo, J., Jones, P. L., Yu, T., Barker, R., Weekes, M. P., & Duraisingh, M. T. (2017). CRISPR/Cas9 knockouts reveal genetic interaction between strain-transcendent erythrocyte determinants of Plasmodium falciparum invasion. Proc Natl Acad Sci U S A, 114(44), E9356-E9365. https://doi.org/10.1073/pnas.1711310114
Kumar, H., & Tolia, N. H. (2019). Getting in: The structural biology of malaria invasion. PLoS Pathog, 15(9), e1007943. https://doi.org/10.1371/journal.ppat.1007943
Maïssa, N., Covarelli, V., Janel, S., Durel, B., Simpson, N., Bernard, S. C., Pardo-Lopez, L., Bouzinba-Ségard, H., Faure, C., Scott, M. G. H., Coureuil, M., Morand, P. C., Lafont, F., Nassif, X., Marullo, S., & Bourdoulous, S. (2017). Strength of Neisseria meningitidis binding to endothelial cells requires highly-ordered CD147/β2-adrenoceptor clusters assembled by alpha-actinin-4. Nat Commun, 8, 15764. https://doi.org/10.1038/ncomms15764
Mousa, A., Al-Taiar, A., Anstey, N. M., Badaut, C., Barber, B. E., Bassat, Q., Challenger, J. D., Cunnington, A. J., Datta, D., Drakeley, C., Ghani, A. C., Gordeuk, V. R., Grigg, M. J., Hugo, P., John, C. C., Mayor, A., Migot-Nabias, F., Opoka, R. O., Pasvol, G., ... Okell, L. C. (2020). The impact of delayed treatment of uncomplicated P. falciparum malaria on progression to severe malaria: A systematic review and a pooled multicentre individual-patient meta-analysis. PLoS Med, 17(10), e1003359. https://doi.org/10.1371/iournal.pmed.1003359
Phillips, M. A., Burrows, J. N., Manyando, C., van Huijsduijnen, R. H., Van Voorhis, W. C., & Wells, T. N. C. (2017). Malaria. Nat Rev Dis Primers, 3, 17050. https://doi.org/10.1038/nrdp.2017.50
Pino, P., Caldelari, R., Mukherjee, B., Vahokoski, J., Klages, N., Maco, B., Collins, C. R., Blackman, M. J., Kursula, I., Heussler, V., Brochet, M., & Soldati-Favre, D. (2017). A multistage antimalarial targets the plasmepsins IX and X essential for invasion and egress. Science, 358(6362), 522-528. https://doi.org/10.1126/science.aaf8675
Reddy, K. S., Amlabu, E., Pandey, A. K., Mitra, P., Chauhan, V. S., & Gaur, D. (2015). Multiprotein complex between the GPI-anchored CyRPA with PfRH5 and PfRipr is crucial for Plasmodium falciparum erythrocyte invasion. Proc Natl Acad Sci USA, 112(4), 1179-1184. https://doi.org/10.1073/pnas.1415466112
Reid, M. E., Lomas-Francis, C., & Olsson, M. L. (2012). OK - Ok blood group system. In M. E. Reid, C. Lomas-Francis, & M. L. Olsson (Eds.), The Blood Group Antigen FactsBook (3rd ed., pp. 577-583). Academic Press.
Satchwell, T. J., Wright, K. E., Haydn-Smith, K. L., Sánchez-Román Terán, F., Moura, P. L., Hawksworth, J., Frayne, J., Toye, A. M., & Baum, J. (2019). Genetic manipulation of cell line derived reticulocytes enables dissection of host malaria invasion requirements. Nat Commun, 10(1), 3806. https://doi.org/10.1038/s41467-019-11790-w
Scally, S. W., Triglia, T., Evelyn, C., Seager, B. A., Pasternak, M., Lim, P. S., Healer, J., Geoghegan, N. D., Adair, A., Tham, W. H., Dagley, L. F., Rogers, K. L., & Cowman, A. F. (2022). PCRCR complex is essential for invasion of human erythrocytes by Plasmodium falciparum. Nat Microbiol, 7(12), 2039-2053. https://doi.org/10.1038/s41564-022-01261-2
Till, A., Rosenstiel, P., Bräutigam, K., Sina, C., Jacobs, G., Oberg, H. H., Seegert, D., Chakraborty, T., & Schreiber, S. (2008). A role for membrane-bound CD147 in NOD2-mediated recognition of bacterial cytoinvasion. J Cell Sci, 121(Pt 4), 487-495. https://doi.org/10.1242/jcs.016980
Triglia, T., Scally, S. W., Seager, B. A., Pasternak, M., Dagley, L. F., & Cowman, A. F. (2023). Plasmepsin X activates the PCRCR complex of Plasmodium falciparum by processing PfRh5 for erythrocyte invasion. Nat Commun, 14(1), 2219. https://doi.org/10.1038/s41467-023-37890-2
von Ungern-Sternberg, S. N. I., Zernecke, A., & Seizer, P. (2018). Extracellular Matrix Metalloproteinase Inducer EMMPRIN (CD147) in Cardiovascular Disease. Int J Mol Sci, 19(2), 507. https://doi.org/10.3390/iims19020507
Wanaguru, M., Liu, W., Hahn, B. H., Rayner, J. C., & Wright, G. J. (2013). PfRH5-Basigin interaction plays a major role in the host tropism of Plasmodium falciparum. Proc Natl Acad Sci USA, 110(51), 20735-20740. https://doi.org/10.1073/pnas.1320771110
Weidle, U. H., Scheuer, W., Eggle, D., Klostermann, S., & Stockinger, H. (2010). Cancer-related issues of CD147. Cancer Genomics Proteomics, 7(3), 157-169. https://pubmed.ncbi.nlm.nih.gov/20551248
Wicht, K. J., Mok, S., & Fidock, D. A. (2020). Molecular Mechanisms of Drug Resistance in Plasmodium falciparum Malaria. Annu Rev Microbiol, 74, 431-454. https://doi.org/10.1146/annurev-micro-020518-115546
Williams, A. R., Douglas, A. D., Miura, K., Illingworth, J. J., Choudhary, P., Murungi, L. M., Furze, J. M., Diouf, A., Miotto, O., Crosnier, C., Wright, G. J., Kwiatkowski, D. P., Fairhurst, R. M., Long, C. A., & Draper, S. J. (2012). Enhancing blockade of Plasmodium falciparum erythrocyte invasion: assessing combinations of antibodies against PfRH5 and other merozoite antigens. PLoS Pathog, 8(11), e1002991. https://doi.org/10.1371/iournal.ppat.1002991
Wright, K. E., Hjerrild, K. A., Bartlett, J., Douglas, A. D., Jin, J., Brown, R. E., Illingworth, J. J., Ashfield, R., Clemmensen, S. B., de Jongh, W. A., Draper, S. J., & Higgins, M. K. (2014). Structure of malaria invasion protein PfRH5 with erythrocyte basigin and blocking antibodies. Nature, 515(7527), 427-430. https://doi.org/10.1038/nature13715
Zavala, F. (2022). RTS,S: the first malaria vaccine. J Clin Invest, 132(1), e156588. https://doi.org/10.1172/JCI156588
Zenonos, Z. A., Dummler, S. K., Müller-Sienerth, N., Chen, J., Preiser, P. R., Rayner, J. C., & Wright, G. J. (2015). Basigin is a druggable target for host-oriented antimalarial interventions. J Exp Med, 212(8), 1145-1151. https://doi.org/10.1084/iem.20150032
Zhu, X., Song, Z., Zhang, S., Nanda, A., & Li, G. (2014). CD147: a novel modulator of inflammatory and immune disorders. Curr Med Chem, 21(19), 2138-2145. https://doi.org/1 0.2174/0929867321666131227163352

## Claims

1. A VHH antibody recognizing basigin.

2. The VHH antibody of claim 1 which binds to the same or an overlapping epitope on basigin as one of the following VHH antibodies:
- the VHH antibody Bm7D12 having a VHH sequence as shown in SEQ. ID NO: 5;
- the VHH antibody Bm8D12 having a VHH sequence as shown in SEQ. ID NO: 37;
- the VHH antibody Bm8G10 having a VHH sequence as shown in SEQ. ID NO: 65;
- the VHH antibody Bm8H10 having a VHH sequence as shown in SEQ. ID NO: 73;
- the VHH antibody Bm8G07 having a VHH sequence as shown in SEQ. ID NO: 81;
- the VHH antibody Bm8G12 having a VHH sequence as shown in SEQ. ID NO: 85;
- the VHH antibody Bm27E06 having a VHH sequence as shown in SEQ. ID NO: 97; or
- the VHH antibody Bm27C11 having a VHH sequence as shown in SEQ. ID NO: 105.

3. The VHH antibody of any of the preceding claims, comprising
(a) a combination of CDR1, CDR2 and CDR3 sequences as shown in SEQ. ID NO: 6-8, 10-12, 14-16, 18-20, 22-24, 26-28, 30-32, 34-36, 38-40, 42-44, 46-48, 50-52, 54-56, 58-60, 62-64, 66-68, 70-72, 74-76, 78-80, 82-84, 86-88, 90-92, 94-96, 98-100, 102-104, 106-108 or 110-112; or
(b) a combination of CDR1, CDR2 and CDR3 sequences which has an identity of at least 80%, at least 90% or at least 95% to a combination of CDR1, CDR2 and CDR3 sequences of (a).

4. The VHH antibody of any one of the preceding claims, comprising
(a) a VHH sequence as shown in SEQ. ID NO: 5, 9, 13, 17, 21, 25, 29, 33, 37, 41, 45, 49, 53, 57, 61, 65, 69, 73, 77, 81, 85, 89, 93, 97, 101, 105, or 109; or
(b) a sequence, which has an identity of at least 80%, at least 90%, at least 95% or at least 99% to a VHH sequence of (a).

5. The VHH antibody of any one of the preceding claims, wherein the binding affinity expressed as dissociation constant K_{D} to basigin is about 500 pM or less, about 100 pM or less, or about 10 pM or less.

6. The VHH antibody of any one of the preceding claims, which inhibits the binding of PfRH5 to erythrocytes and which inhibits the entry of Plasmodia into erythrocytes.

7. The VHH antibody of any one of the preceding claims, which has:
- a melting temperature of at least about 60°C, of at least 80°C or of at least about 95°C when measured under non-reducing conditions; and/or
- a melting temperature of at least about 30°C, of at least about 50°C or of at least about 95°C when measured under reducing conditions.

8. The VHH antibody of any one of preceding claims, which is selected from;
- VHH antibody Bm7D12 having a VHH sequence as shown in SEQ. ID NO: 5 or a VHH antibody, which is a variant thereof,
- VHH antibody Bm7H08 having a VHH sequence as shown in SEQ. ID NO: 9 or a VHH antibody, which is a variant thereof,
- VHH antibody Bm7G12 having a VHH sequence as shown in SEQ. ID NO: 13 or a VHH antibody, which is a variant thereof,
- VHH antibody Bm7E11 having a VHH sequence as shown in SEQ. ID NO: 17 or a VHH antibody, which is a variant thereof,
- VHH antibody Bm7F03 having a VHH sequence as shown in SEQ. ID NO: 21 or a VHH antibody, which is a variant thereof,
- VHH antibody Bm7G08 having a VHH sequence as shown in SEQ. ID NO: 25 or a VHH antibody, which is a variant thereof,
- VHH antibody Bm7D04 having a VHH sequence as shown in SEQ. ID NO: 29 or a VHH antibody, which is a variant thereof,
- VHH antibody Bm7H02 having a VHH sequence as shown in SEQ. ID NO: 33 or a VHH antibody, which is a variant thereof,
- VHH antibody Bm8D12 having a VHH sequence as shown in SEQ. ID NO: 37 or a VHH antibody, which is a variant thereof,
- VHH antibody Bm8B10 having a VHH sequence as shown in SEQ. ID NO: 41 or a VHH antibody, which is a variant thereof,
- VHH antibody Bm8D09 having a VHH sequence as shown in SEQ. ID NO: 45 or a VHH antibody, which is a variant thereof,
- VHH antibody Bm8H12 having a VHH sequence as shown in SEQ. ID NO: 49 or a VHH antibody, which is a variant thereof,
- VHH antibody Bm8C06 having a VHH sequence as shown in SEQ. ID NO: 53 or a VHH antibody, which is a variant thereof,
- VHH antibody Bm8H07 having a VHH sequence as shown in SEQ. ID NO: 57 or a VHH antibody, which is a variant thereof,
- VHH antibody Bm8G09 having a VHH sequence as shown in SEQ. ID NO: 61 or a VHH antibody, which is a variant thereof,
- VHH antibody Bm8G10 having a VHH sequence as shown in SEQ. ID NO: 65 or a VHH antibody, which is a variant thereof,
- VHH antibody Bm8F05 having a VHH sequence as shown in SEQ. ID NO: 69 or a VHH antibody, which is a variant thereof,
- VHH antibody Bm8H10 having a VHH sequence as shown in SEQ. ID NO: 73 or a VHH antibody, which is a variant thereof,
- VHH antibody Bm8G06 having a VHH sequence as shown in SEQ. ID NO: 77 or a VHH antibody, which is a variant thereof,
- VHH antibody Bm8G07 having a VHH sequence as shown in SEQ. ID NO: 81 or a VHH antibody, which is a variant thereof,
- VHH antibody Bm8G12 having a VHH sequence as shown in SEQ. ID NO: 85 or a VHH antibody, which is a variant thereof,
- VHH antibody Bm27C04 having a VHH sequence as shown in SEQ. ID NO: 89 or a VHH antibody, which is a variant thereof,
- VHH antibody Bm27C06 having a VHH sequence as shown in SEQ. ID NO: 93 or a VHH antibody, which is a variant thereof,
- VHH antibody Bm27E06 having a VHH sequence as shown in SEQ. ID NO: 97 or a VHH antibody, which is a variant thereof,
- VHH antibody Bm27H03 having a VHH sequence as shown in SEQ. ID NO: 101 or a VHH antibody, which is a variant thereof,
- VHH antibody Bm27C11 having a VHH sequence as shown in SEQ. ID NO: 105 or a VHH antibody, which is a variant thereof, and
- VHH antibody Bm27D06 having a VHH sequence as shown in SEQ. ID NO: 109 or a VHH antibody, which is a variant thereof.

9. A set of two or more different VHH antibodies recognizing basigin, comprising at least one VHH antibody of any one of the preceding claims.

10. A pharmaceutical composition comprising as an active agent the VHH antibody of any one of claims 1-8 or the set of claim 9, and a pharmaceutically acceptable carrier.

11. The VHH antibody of any one of claims 1-8, the set of claim 9 or the pharmaceutical composition of claim 10 for use as a medicament, particularly in human medicine.

12. The VHH antibody of any one of claims 1-8, the set of claim 9 or the pharmaceutical composition of claim 10 for use in the prevention, treatment and/or mitigation of a condition caused by, associated with and/or accompanied by an activity of basigin.

13. The VHH antibody of any one of claims 1-8, the set of claim 9 or the pharmaceutical composition of claim 10 for use in the prevention, treatment and/or mitigation of a condition caused by, associated with and/or accompanied by an infection with a pathogen that invades mammalian cells by interaction with basigin, particularly for the prevention, treatment and/or mitigation of malaria.

14. Use of the VHH antibody of any one of claims 1-8 or the set of claim 9 for detecting basigin in a sample, particularly in a biological sample, e.g., a body fluid such as saliva, sputum, a lung lavage, a swab, blood, serum or plasma, a stool sample, a tissue sample, or a biopsy sample.

15. A method for the prevention, treatment and/or mitigation of a condition caused by, associated with and/or accompanied by an activity of basigin comprising administering an effective dose of the VHH antibody of any one of claims 1-8, the set of claim 9 or the pharmaceutical composition of claim 10 to a subject in need thereof, particularly to a human subject.
